# EUROPÄISCHE PATENTSCHRIFT

(11) **EP 3 527 661 B1**
(45) Veröffentlichungstag und Bekanntmachung des Hinweises auf die Patenterteilung: **01.12.2021**
(21) Anmeldenummer: 18212311.7
(22) Anmeldetag: 25.05.2012
(51) Int. Cl.: C12N 15/00

(54) **EXPRESSIONSVEKTOREN ZUR VERBESSERTEN PROTEINSEKRETION**
EXPRESSION VECTORS FOR ENHANCED PROTEIN SECRETION
VECTEURS D'EXPRESSION DESTINÉS À LA SÉCRÉTION DE PROTEINES AMÉLIORÉE

(30) Priorität: 31.05.2011 DE 102011118032
(43) Veröffentlichungstag der Anmeldung: 21.08.2019
(62) Teilanmeldung aus: 16178440.0
(73) Patentinhaber: BASF SE, 67056 Ludwigshafen am Rhein (DE)
(72) Erfinder: DEGERING, Christian, 40699 Erkrath (DE); EGGERT, Thorsten, 45529 Hattingen (DE); EVERS, Stefan, 42781 Haan (DE); MAURER, Karl-Heinz, 40699 Erkrath (DE); BONGAERTS, Johannes, 52428 Juelich (DE)
(74) Vertreter: BASF IP Association

(56) Entgegenhaltungen:
- WO-A2-2008/066931

### SEQUENCE LISTING

<110> BASF SE
<120> Expressionsvektoren zur verbesserten Proteinsekretion
<130> 0000075519EP03
<150> DE 102011118032.3
   <151> 2011-05-31
<160> 22
<170> PatentIn version 3.5
<210> 1
   <211> 126
   <212> DNA
   <213> Bacillus subtilis
<220>
   <221> CDS
   <222> (1)..(126)
<400> 1
   atg gcg aaa cca cta tca aaa ggg gga att ttg gtg aaa aaa gta ttg         48
   Met Ala Lys Pro Leu Ser Lys Gly Gly Ile Leu Val Lys Lys Val Leu
   1               5                   10                  15
   att gca ggt gca gta gga aca gca gtt ctt ttc gga acc ctt tca tca         96
   Ile Ala Gly Ala Val Gly Thr Ala Val Leu Phe Gly Thr Leu Ser Ser
               20                  25                  30
   ggt ata cca ggt tta ccc gcg gca gac gct                                126
   Gly Ile Pro Gly Leu Pro Ala Ala Asp Ala
           35                  40
<210> 2
   <211> 42
   <212> PRT
   <213> Bacillus subtilis
<400> 2
   Met Ala Lys Pro Leu Ser Lys Gly Gly Ile Leu Val Lys Lys Val Leu
   1               5                   10                  15
   Ile Ala Gly Ala Val Gly Thr Ala Val Leu Phe Gly Thr Leu Ser Ser
               20                  25                  30
   Gly Ile Pro Gly Leu Pro Ala Ala Asp Ala
           35                  40
<210> 3
   <211> 81
   <212> DNA
   <213> Bacillus licheniformis
<220>
   <221> CDS
   <222> (1)..(81)
<400> 3
   atg aaa aaa cta ttc aaa acc att gtt aca ctg tca ctc ttg att tct         48
   Met Lys Lys Leu Phe Lys Thr Ile Val Thr Leu Ser Leu Leu Ile Ser
   1               5                   10                  15
   gga acg ctt tta ttc tca caa tct gcg gcg gct                             81
   Gly Thr Leu Leu Phe Ser Gln Ser Ala Ala Ala
               20                  25
<210> 4
   <211> 27
   <212> PRT
   <213> Bacillus licheniformis
<400> 4
   Met Lys Lys Leu Phe Lys Thr Ile Val Thr Leu Ser Leu Leu Ile Ser
   1               5                   10                  15
   Gly Thr Leu Leu Phe Ser Gln Ser Ala Ala Ala
               20                  25
<210> 5
   <211> 84
   <212> DNA
   <213> Bacillus subtilis
<220>
   <221> CDS
   <222> (1)..(84)
<400> 5
   atg aca ttg act aaa ctg aaa atg ctg agt atg tta acc gtg atg att         48
   Met Thr Leu Thr Lys Leu Lys Met Leu Ser Met Leu Thr Val Met Ile
   1               5                   10                  15
   gca tct tta ttc att ttt tcc agt cag gca ctt gcc                         84
   Ala Ser Leu Phe Ile Phe Ser Ser Gln Ala Leu Ala
               20                  25
<210> 6
   <211> 28
   <212> PRT
   <213> Bacillus subtilis
<400> 6
   Met Thr Leu Thr Lys Leu Lys Met Leu Ser Met Leu Thr Val Met Ile
   1               5                   10                  15
   Ala Ser Leu Phe Ile Phe Ser Ser Gln Ala Leu Ala
                       20                  25
<210> 7
   <211> 269
   <212> PRT
   <213> Bacillus lentus
<400> 7
   Ala Gln Thr Ile Pro Trp Gly Ile Ser Arg Val Gln Ala Pro Ala Ala
   1               5                   10                  15
   His Asn Arg Gly Leu Thr Gly Ser Gly Val Lys Val Ala Val Leu Asp
               20                  25                  30
   Thr Gly Ile Ser Thr His Pro Asp Leu Asn Ile Arg Gly Gly Ala Ser
           35                  40                  45
   Phe Val Pro Gly Glu Pro Ser Thr Gln Asp Gly Asn Gly His Gly Thr
       50                  55                  60
   His Val Ala Gly Thr Ile Ala Ala Leu Asn Asn Ser Ile Gly Val Leu
   65                  70                  75                  80
   Gly Val Ala Pro Ser Ala Glu Leu Tyr Ala Val Lys Val Leu Gly Ala
                   85                  90                  95
   Asp Gly Arg Gly Ala Ile Ser Ser Ile Ala Gln Gly Leu Glu Trp Ala
               100                 105                 110
   Gly Asn Asn Gly Met His Val Ala Asn Leu Ser Leu Gly Ser Pro Ser
           115                 120                 125
   Pro Ser Ala Thr Leu Glu Gln Ala Val Asn Ser Ala Thr Ser Arg Gly
       130                 135                 140
   Val Leu Val Val Ala Ala Ser Gly Asn Ser Gly Ala Ser Ser Ile Ser
   145                 150                 155                 160
   Tyr Pro Ala Arg Tyr Ala Asn Ala Met Ala Val Gly Ala Thr Asp Gln
                   165                 170                 175
   Asn Asn Asn Arg Ala Ser Phe Ser Gln Tyr Gly Ala Gly Leu Asp Ile
               180                 185                 190
   Val Ala Pro Gly Val Asn Ile Gln Ser Thr Tyr Pro Gly Ser Thr Tyr
           195                 200                 205
   Ala Ser Leu Asn Gly Thr Ser Met Ala Thr Pro His Val Ala Gly Ala
       210                 215                 220
   Ala Ala Leu Val Lys Gln Lys Asn Pro Ser Trp Ser Asn Val Gln Ile
   225                 230                 235                 240
   Arg Asn His Leu Lys Asn Thr Ala Thr Ser Leu Gly Ser Thr Asn Leu
                   245                 250                 255
   Tyr Gly Ser Gly Leu Val Asn Ala Glu Ala Ala Thr Arg
               260                 265
<210> 8
   <211> 269
   <212> PRT
   <213> Bacillus lentus
<400> 8
   Ala Gln Thr Ile Pro Trp Gly Ile Ser Arg Val Gln Ala Pro Ala Ala
   1 5 10 15
   His Asn Arg Gly Leu Thr Gly Ser Gly Val Lys Val Ala Val Leu Asp
   20 25 30
   Thr Gly Ile Ser Thr His Pro Asp Leu Asn Ile Arg Gly Gly Ala Ser
   35 40 45
   Phe Val Pro Gly Glu Pro Ser Thr Gln Asp Gly Asn Gly His Gly Thr
   50 55 60
   His Val Ala Gly Thr Ile Ala Ala Leu Asn Asn Ser Ile Gly Val Leu
   65 70 75 80
   Gly Val Ala Pro Ser Ala Glu Leu Tyr Ala Val Lys Val Leu Gly Ala
   85 90 95
   Asp Gly Arg Gly Ala Ile Ser Ser Ile Ala Gln Gly Leu Glu Trp Ala
   100 105 110
   Gly Asn Asn Gly Met His Val Ala Asn Leu Ser Leu Gly Ser Pro Ser
   115 120 125
   Pro Ser Ala Thr Leu Glu Gln Ala Val Asn Ser Ala Thr Ser Arg Gly
   130 135 140
   Val Leu Val Val Ala Ala Ser Gly Asn Ser Gly Ala Ser Ser Ile Ser
   145 150 155 160
   Tyr Pro Ala Arg Tyr Ala Asn Ala Met Ala Val Gly Ala Thr Asp Gln
                   165                 170                 175
   Asn Asn Asn Arg Ala Ser Phe Ser Gln Tyr Gly Ala Gly Leu Asp Ile
               180                 185                 190
   Met Ala Pro Gly Val Asn Ile Gln Ser Thr Tyr Pro Gly Ser Thr Tyr
           195                 200                 205
   Ala Ser Asp Asn Gly Thr Ser Met Ala Thr Pro His Val Ala Gly Ala
       210                 215                 220
   Ala Ala Leu Val Lys Gln Lys Asn Pro Ser Trp Ser Asn Val Gln Ile
   225                 230                 235                 240
   Arg Asn His Leu Lys Asn Thr Ala Thr Ser Leu Gly Ser Thr Asn Leu
                   245                 250                 255
   Tyr Gly Ser Gly Leu Val Asn Ala Glu Ala Ala Thr Arg
               260                 265
<210> 9
   <211> 269
   <212> PRT
   <213> Bacillus gibsonii
<400> 9
   Gln Gln Thr Val Pro Trp Gly Ile Thr Arg Val Gln Ala Pro Thr Val
   1               5                   10                  15
   His Asn Arg Gly Ile Thr Gly Ser Gly Val Lys Val Ala Ile Leu Asp
               20                  25                  30
   Thr Gly Ile Ala Gln His Ser Asp Leu Thr Ile Arg Gly Gly Ala Ser
           35                  40                  45
   Phe Val Pro Gly Glu Ser Thr Thr Ala Asp Leu Asn Gly His Gly Thr
       50                  55                  60
   His Val Ala Gly Thr Val Ala Ala Leu Asn Asn Ser Ile Gly Val Ile
   65                  70                  75                  80
   Gly Val Ala Pro Ser Ala Asp Leu Tyr Ala Val Lys Val Leu Gly Ala
                   85                  90                  95
   Asn Gly Arg Gly Ser Val Ser Gly Ile Ala Gln Gly Leu Glu Trp Ala
               100                 105                 110
   Ala Thr Asn Asn Met His Ile Ala Asn Met Ser Leu Gly Ser Asp Ala
                   115                 120                 125
   Pro Ser Thr Thr Leu Glu Arg Ala Val Asn Tyr Ala Thr Ser Arg Gly
       130                 135                 140
   Val Leu Val Ile Ala Ala Thr Gly Asn Asn Gly Thr Gly Ser Ile Gly
   145                 150                 155                 160
   Tyr Pro Ala Arg Tyr Ala Asn Ala Met Ala Val Gly Ala Thr Asp Gln
                   165                 170                 175
   Asn Asn Arg Arg Ala Ser Phe Ser Gln Tyr Gly Thr Gly Ile Asp Ile
               180                 185                 190
   Val Ala Pro Gly Val Gly Ile Gln Ser Thr Tyr Leu Asn Asn Ser Tyr
           195                 200                 205
   Ala Ser Met Pro Gly Thr Ser Met Ala Thr Pro His Val Ala Gly Val
       210                 215                 220
   Ala Ala Leu Val Lys Gln Lys Asn Pro Ser Trp Asn Ala Thr Gln Ile
   225                 230                 235                 240
   Arg Asn His Leu Lys Asn Thr Ala Thr Asn Leu Gly Asn Ser Ser Gln
                   245                 250                 255
   Phe Gly Ser Gly Leu Val Asn Ala Asp Ala Ala Thr Arg
               260                 265
<210> 10
   <211> 269
   <212> PRT
   <213> Bacillus lentus
<400> 10
   Ala Gln Ser Val Pro Trp Gly Ile Ser Arg Val Gln Ala Pro Ala Ala
   1               5                   10                  15
   His Asn Arg Gly Leu Thr Gly Ser Gly Val Lys Val Ala Val Leu Asp
               20                  25                  30
   Thr Gly Ile Ser Thr His Pro Asp Leu Asn Ile Arg Gly Gly Ala Ser
           35                  40                  45
   Phe Val Pro Gly Glu Pro Ser Thr Gln Asp Gly Asn Gly His Gly Thr
       50                  55                  60
   His Val Ala Gly Thr Ile Ala Ala Leu Asn Asn Ser Ile Gly Val Leu
   65                  70                  75                  80
   Gly Val Ala Pro Ser Ala Glu Leu Tyr Ala Val Lys Val Leu Gly Ala
                   85                  90                  95
   Asp Gly Arg Gly Ala Ile Ser Ser Ile Ala Gln Gly Leu Glu Trp Ala
               100                 105                 110
   Gly Asn Asn Gly Met His Val Ala Asn Leu Ser Leu Gly Ser Pro Ser
           115                 120                 125
   Pro Ser Ala Thr Leu Glu Gln Ala Val Asn Ser Ala Thr Ser Arg Gly
       130                 135                 140
   Val Leu Val Val Ala Ala Ser Gly Asn Ser Gly Ala Ser Ser Ile Ser
   145                 150                 155                 160
   Tyr Pro Ala Arg Tyr Ala Asn Ala Met Ala Val Gly Ala Thr Asp Gln
                   165                 170                 175
   Asn Asn Asn Arg Ala Ser Phe Ser Gln Tyr Gly Ala Gly Leu Asp Ile
               180                 185                 190
   Val Ala Pro Gly Val Asn Val Gln Ser Thr Tyr Pro Gly Ser Thr Tyr
           195                 200                 205
   Ala Ser Leu Asn Gly Thr Ser Met Ala Thr Pro His Val Ala Gly Ala
       210                 215                 220
   Ala Ala Leu Val Lys Gln Lys Asn Pro Ser Trp Ser Asn Val Gln Ile
   225                 230                 235                 240
   Arg Asn His Leu Lys Asn Thr Ala Thr Ser Leu Gly Ser Thr Asn Leu
                   245                 250                 255
   Tyr Gly Ser Gly Leu Val Asn Ala Glu Ala Ala Thr Arg
               260                 265
<210> 11
   <211> 30
   <212> DNA
   <213> Artificial
<220>
   <223> upstream primer
<400> 11
   atatgaattc gctgaggaag caaaagaaaa 30
<210> 12
   <211> 31
   <212> DNA
   <213> Artificial
<220>
   <223> downstream primer
<400> 12
   atatggatcc ttagcgtgtt gccgcttctg c 31
<210> 13
   <211> 30
   <212> DNA
   <213> Artificial
<220>
   <223> upstream primer
<400> 13
   atatgaattc gctgaggaag caaaagaaaa 30
<210> 14
   <211> 31
   <212> DNA
   <213> Artificial
<220>
   <223> downstream primer
<400> 14
   atatggatcc ttagcgcgtt gctgcatctg c 31
<210> 15
   <211> 43
   <212> DNA
   <213> Artificial
<220>
   <223> upstream primer
<400> 15
   atataagctt aaggaggata ttatgatgag gaaaaagagt ttt 43
<210> 16
   <211> 34
   <212> DNA
   <213> Artificial
<220>
   <223> downstream primer
<400> 16
   atatgaattc agctgcagaa gcggaatcgc tgaa 34
<210> 17
   <211> 43
   <212> DNA
   <213> Artificial
<220>
   <223> upstream primer
<400> 17
   atataagctt aaggaggata ttatgaaaaa actattcaaa acc 43
<210> 18
   <211> 34
   <212> DNA
   <213> Artificial
<220>
   <223> downstream primer
<400> 18
   atatgaattc agcagccgcc gcagattgtg agaa 34
<210> 19
   <211> 43
   <212> DNA
   <213> Artificial
<220>
   <223> upstream primer
<400> 19
   atataagctt aaggaggata ttatggcgaa accactatca aaa 43
<210> 20
   <211> 34
   <212> DNA
   <213> Artificial
<220>
   <223> downstream primer
<400> 20
   atatgaattc agcagcgtct gccgcgggta aacc 34
<210> 21
   <211> 43
   <212> DNA
   <213> Artificial
<220>
   <223> upstream primer
<400> 21
   atataagctt aaggaggata ttatgacatt gactaaactg aaa 43
<210> 22
   <211> 34
   <212> DNA
   <213> Artificial
<220>
   <223> downstream primer
<400> 22
   atatgaattc agcggcaagt gcctgactgg aaaa 34

## Beschreibung

Die Erfindung liegt auf dem Gebiet der Biotechnologie, insbesondere der mikrobiellen Proteinsynthese. Die Erfindung betrifft insbesondere Expressionsvektoren zur Herstellung von Proteinen und schlägt ferner Wirtszellen vor, die derartige Expressionsvektoren enthalten. Die Erfindung betrifft ferner Verfahren und Verwendungen derartiger Expressionsvektoren und Wirtszellen zur Proteinherstellung.

Für die Herstellung von Proteinen können Wirtszellen, insbesondere Mikroorganismen, eingesetzt werden, die die Gene der interessierenden Proteine exprimieren. Das Gen eines Proteins von Interesse (Transgen) wird in der Regel in die Wirtszellen derart eingebracht, dass es von diesen exprimiert wird. Häufig liegt es auf einem so genannten Expressionsvektor zusammen mit einer oder mehreren Promotorsequenzen (Promotoren) vor, wodurch die Genexpression ermöglicht wird.

Für die großtechnische, biotechnologische Produktion werden die betreffenden Wirtszellen in Fermentern kultiviert, die den Stoffwechseleigenschaften der Zellen entsprechend ausgestaltet sind. Während der Kultivierung verstoffwechseln die Wirtszellen das angebotene Substrat und bilden das gewünschte Produkt, das nach Beendigung der Fermentation üblicherweise von den Produktionsorganismen abgetrennt wird und aus dem Fermenterbrei und/oder dem Fermentationsmedium aufgereinigt und/oder aufkonzentriert wird.

Es ist grundsätzlich wünschenswert, eine möglichst hohe Produktausbeute in der Fermentation zu erhalten. Die Produktausbeute ist dabei abhängig von mehreren Faktoren, beispielsweise bilden die Wirtszellen neben dem eigentlich gewünschten Produkt üblicherweise eine Vielzahl weiterer Substanzen, an denen in der Regel kein Interesse besteht. Weiter hängt die Expression eines Transgens und damit die Produktausbeute wesentlich von dem verwendeten Expressionssystem ab. Beispielsweise offenbart die internationale Patentanmeldung WO 91/02792 die verbesserte fermentative Produktion einer alkalischen Protease aus Bacillus lentus in einem optimierten Bacillus licheniformis-Stamm unter der Kontrolle genregulatorischer Sequenzen aus Bacillus licheniformis, insbesondere des Bacillus licheniformis-Promotors.

Vorzugsweise werden für die industrielle Produktion von Proteinen, beispielsweise hydrolytischen Enzymen, solche Wirtszellen eingesetzt, die in der Lage sind große Mengen des Proteins in den Kulturüberstand zu sezernieren, was einen aufwändigen Zellaufschluss, der bei der intrazellulären Produktion notwendig ist, überflüssig macht. Hierfür werden vorzugsweise solche Wirtszellen, beispielsweise Bacillus-Spezies, eingesetzt, die sich mit kostengünstigen Nährmedien in effizienten Hochzelldichte-Fermentationen kultivieren lassen und in der Lage sind, mehrere Gramm pro Liter des Zielproteins in den Kulturüberstand zu sezernieren. Üblicherweise wird das zu sezernierende Protein von Expressionsvektoren exprimiert, die in die Wirtszelle eingebracht worden sind und für das zu sezernierende Protein codieren. Das exprimierte Protein umfasst üblicherweise ein Signalpeptid (Signalsequenz), die dessen Export aus der Wirtszelle bewirkt. Das Signalpeptid ist üblicherweise Teil der in der Wirtszelle translatierten Polypeptidkette, es kann von dem Protein aber posttranslational noch innerhalb oder außerhalb der Wirtszelle abgespalten werden.

Gerade für diese extrazelluläre Produktion von heterologen Proteinen gibt es allerdings zahlreiche Engpässe und einen dementsprechend hohen Bedarf, die Abläufe der Sekretion zu optimieren. Einer dieser Engpässe ist die Auswahl eines Signalpeptids, das einen effizienten Export des Zielproteins aus der Wirtszelle erlaubt. Signalpeptide können grundsätzlich neu kombiniert werden mit Proteinen, insbesondere Enzymen. Beispielsweise wird in der Veröffentlichung von Brockmeier et al. (J. Mol. Biol. 362, S. 393-402 (2006)) die Strategie des Screenings einer Signalpeptid-Bibliothek am Beispiel einer Cutinase beschrieben. Jedoch bewirkt nicht jedes Signalpeptid auch einen ausreichenden Export des Proteins unter Fermentationsbedingungen, insbesondere industriellen bzw. großtechnischen Fermentationsbedingungen.

WO2008066931 offenbart die Isolierung des kompletten Chromosoms von B.licheniformis SJ1904.

Aufgabe der Erfindung ist es daher, die Sekretion eines Proteins aus einer Wirtszelle zu verbessern und dadurch die Produktausbeute an Protein in einer Fermentation zu steigern, wobei das Protein eine Protease, Amylase, Cellulase, Hemicellulase, Mannanase, Tannase, Xylanase, Xanthanase, Xyloglucanase, ß-Glucosidase, Pektin-spaltendes Enzym, Carrageenase, Perhydrolase, Oxidase, Oxidoreduktase oder eine Lipase ist.

Gegenstand der Erfindung ist ein Expressionsvektor umfassend
a) eine Promotorsequenz und
b) eine Nukleinsäuresequenz, die für ein Protein codiert, wobei das Protein ein Signalpeptid und eine weitere Aminosäuresequenz umfasst und das Signalpeptid eine Aminosäuresequenz umfasst, die zu der in SEQ ID NO. 4 angegebenen Aminosäuresequenz zu mindestens 80% identisch ist,, dadurch gekennzeichnet, dass die weitere Aminosäuresequenz des Proteins die Aminosäuresequenz einer Protease, Amylase, Cellulase, Hemicellulase, Mannanase, Tannase, Xylanase, Xanthanase, Xyloglucanase, ß-Glucosidase, Pektin-spaltendes Enzym, Carrageenase, Perhydrolase, Oxidase, Oxidoreduktase oder einer Lipase umfasst.

Überraschenderweise wurde gefunden, dass durch einen Expressionsvektor, der für ein Protein mit einem derartigen Signalpeptid codiert, eine verbesserte Sekretion des Proteins aus einer Wirtszelle erreicht wird, die den Expressionsvektor beinhaltet und die Nukleinsäuresequenz b) exprimiert. Hierdurch ist es in bevorzugten Ausgestaltungen der Erfindung möglich, die Produktausbeute an Protein in einer Fermentation zu steigern.

Ein Expressionsvektor ist eine Nukleinsäuresequenz, die bewirkt, dass das Protein in einer Wirtszelle, insbesondere einem Mikroorganismus, exprimiert werden kann. Er umfasst die genetische Information, also diejenige Nukleinsäuresequenz (Gen) b), die für das Protein codiert.

Die Expression einer Nukleinsäuresequenz ist dessen Übersetzung in das bzw. die von dieser Sequenz codierte(n) Genprodukt(e), also in ein Polypeptid (Protein) bzw. in mehrere Polypeptide (Proteine). Die Begriffe Polypeptid und Protein werden in der vorliegenden Anmeldung synonym verwendet. Im Sinne der vorliegenden Erfindung bezeichnet Expression folglich die Biosynthese von Ribonucleinsäure (RNA) und Proteinen aus den genetischen Informationen. In der Regel umfasst die Expression die Transkription, also die Synthese einer Boten ("messenger")-Ribonukleinsäure (mRNA) anhand der DNA (Desoxyribonukleinsäure)-Sequenz des Gens und deren Translation in die entsprechende Polypeptidkette, die gegebenenfalls noch post-translational modifiziert werden kann. Das Exprimieren eines Proteins beschreibt folglich die Biosynthese desselben aus den genetischen Informationen, die erfindungsgemäß auf dem Expressionsvektor bereitgestellt werden.

Vektoren sind aus Nukleinsäuren, vorzugsweise Desoxyribonukleinsäure (DNA) bestehende genetische Elemente und sind dem Fachmann auf dem Gebiet der Biotechnologie bekannt. Sie sind insbesondere bei der Verwendung in Bakterien spezielle Plasmide, also zirkulare genetische Elemente. Zu den Vektoren können beispielsweise solche gehören, die sich von bakteriellen Plasmiden, von Viren oder von Bacteriophagen ableiten, oder überwiegend synthetische Vektoren oder Plasmide mit Elementen verschiedenster Herkunft. Mit den weiteren jeweils vorhandenen genetischen Elementen vermögen Vektoren sich in Wirtszellen, in die sie vorzugsweise durch Transformation eingebracht wurden, über mehrere Generationen hinweg als stabile Einheiten zu etablieren. Es ist dabei im Sinne der Erfindung unerheblich, ob sie sich extrachomosomal als eigene Einheiten etablieren oder in ein Chromosom bzw. chromosomale DNA integrieren. Welches der zahlreichen Systeme gewählt wird, hängt vom Einzelfall ab. Ausschlaggebend können beispielsweise die erreichbare Kopienzahl, die zur Verfügung stehenden Selektionssysteme, darunter vor allem die Antibiotikaresistenzen, oder die Kultivierbarkeit der zur Aufnahme der Vektoren befähigten Wirtszellen sein.

Expressionsvektoren können ferner durch Änderungen der Kulturbedingungen wie beispielsweise die Zelldichte oder die Zugabe von bestimmten Verbindungen regulierbar sein. Ein Beispiel für eine solche Verbindung ist das Galactose-Derivat Isopropyl-β-D-thiogalactopyranosid (IPTG), welches als Aktivator des bakteriellen Lactose-Operons (lac-Operons) verwendet wird.

Ein Expressionsvektor umfasst ferner mindestens eine Nukleinsäuresequenz, vorzugsweise DNA, mit einer Steuerungsfunktion für die Expression der für das Protein codierenden Nukleinsäuresequenz b) (eine sog. genregulatorische Sequenz). Eine genregulatorische Sequenz ist hierbei jede Nukleinsäuresequenz, deren Anwesenheit in der jeweiligen Wirtszelle die Transkriptionshäufigkeit der Nukleinsäuresequenz b) beeinflusst, vorzugsweise erhöht, die für das Protein codiert. Vorzugsweise handelt es sich um eine Promotor-Sequenz, da eine derartige Sequenz für die Expression der Nukleinsäuresequenz b) wesentlich ist. Ein erfindungsgemäßer Expressionsvektor kann aber auch noch weitere genregulatorische Sequenzen umfassen, beispielsweise eine oder mehrere Enhancer-Sequenzen. Ein Expressionsvektor im Rahmen der Erfindung umfasst folglich mindestens eine funktionelle Einheit aus der Nukleinsäuresequenz b) und einem Promotor (Expressionskassette). Sie kann, muss jedoch nicht notwendigerweise, als physische Einheit vorliegen. Der Promotor bewirkt die Expression der Nukleinsäuresequenz b) in der Wirtszelle. Ein Expressionsvektor kann im Rahmen der vorliegenden Erfindung auch auf die reine Expressionskassette aus Promotor und zu exprimierender Nukleinsäuresequenz b) beschränkt sein, wobei diese Expressionskassette extrachromosomal oder auch chromosomal integriert vorliegen kann. Derartige Ausgestaltungen erfindungsgemäßer Expressionsvektoren stellen jeweils eine gesonderte Ausführungsform der Erfindung dar.

Das Vorhandensein von mindestens einem Promotor ist für einen erfindungsgemäßen Expressionsvektor folglich wesentlich. Unter einem Promotor wird demnach eine DNA-Sequenz verstanden, die die regulierte Expression eines Gens ermöglicht. Natürlicherweise ist eine Promotorsequenz ein Bestandteil eines Gens und liegt oftmals an dessen 5'-Ende und somit vor dem RNA-kodierenden Bereich. Vorzugsweise liegt die Promotorsequenz in einem erfindungsgemäßen Expressionsvektor 5'-wärts von der für das Protein codierenden Nuleinsäuresequenz b). Die wichtigste Eigenschaft eines Promotors ist die spezifische Wechselwirkung mit mindestens einem DNA-bindenden Protein bzw. Polypeptid, welches den Start der Transkription des Gens durch eine RNA-Polymerase vermittelt und als Transkriptionsfaktor bezeichnet wird. Häufig sind mehrere Transkriptionsfaktoren und/oder weitere Proteine am Start der Transkription durch eine RNA-Polymerase beteiligt. Ein Promotor ist demnach vorzugsweise eine DNA-Sequenz mit Promotoraktivität, d.h. eine DNA-Sequenz, an die mindestens ein Transkriptionsfaktor zur Initiation der Transkription eines Gens zumindest transient bindet. Die Stärke eines Promotors ist messbar über die Transkriptionshäufigkeit des exprimierten Gens, also über die Anzahl der pro Zeiteinheit erzeugten RNA-Moleküle, insbesondere mRNA-Moleküle.

Bevorzugt liegen die Promotorsequenz (a) und die Nukleinsäuresequenz (b) hintereinander auf dem Expressionsvektor vor. Weiter bevorzugt befindet sich die Promotorsequenz (a) vor der Nukleinsäuresequenz (b) auf dem Nukleinsäuremolekül (in 5' → 3'-Orientierung). Ebenfalls bevorzugt befinden sich zwischen den beiden Nukleinsäuresequenzen (a) und (b) keine Nukleinsäuresequenzen, die die Transkriptionshäufigkeit der für das Protein codierenden Nukleinsäuresequenz (b) vermindern. Alle vorstehenden Angaben beziehen sich auf denjenigen DNA-Strang, der die für das Protein codierende Nukleinsäuresequenz (b) beinhaltet (den codierenden Strang) und nicht auf den zugehörigen codogenen DNA-Strang. Ausgehend von der für das Protein codierenden Nukleinsäuresequenz (b) befindet sich die Promotorsequenz (a) folglich vorzugsweise weiter stromaufwärts, d.h. in 5'-Richtung, auf diesem DNA-Strang.

Die Nukleinsäuresequenz b) codiert für das zu sezernierende Protein. Hierbei handelt es sich um dasjenige Protein, das mit Hilfe eines erfindungsgemäßen Expressionsvektors hergestellt werden soll (Zielprotein).

Das von der Nukleinsäuresequenz b) codierte Protein umfasst ein Signalpeptid mit einer Aminosäuresequenz, die zu der in SEQ ID NO. 4 angegebenen Aminosäuresequenz zu mindestens 80% identisch ist. Es wurde festgestellt, dass derartige Signalpeptide eine effiziente Sekretion des sie enthaltenden Proteins, insbesondere rekombinanten Proteins, bewirken. Zunehmend bevorzugt umfasst das Signalpeptid eine Aminosäuresequenz, die zu der in SEQ ID NO. 4 angegebenen Aminosäuresequenz zu mindestens 81%, 82%, 83%, 84%, 85%, 86%, 87%, 88%, 89%, 90%, 91%, 92%, 93%, 94%, 95%, 96%, 97%, 98%, 99% und ganz besonders bevorzugt zu 100% identisch ist. Besonders bevorzugt weist das Signalpeptid eine Aminosäuresequenz auf, die zu der in SEQ ID NO. 4 angegebenen Aminosäuresequenz zu mindestens 80%, 81%, 82%, 83%, 84%, 85%, 86%, 87%, 88%, 89%, 90%, 91%, 92%, 93%, 94%, 95%, 96%, 97%, 98%, 99% und ganz besonders bevorzugt zu 100% identisch ist.

Ganz besonders bevorzugt sind jeweils die zu 100% identischen Sequenzen, so dass ein entsprechend bevorzugter Expressionsvektor dadurch gekennzeichnet ist, dass das von der Nukleinsäuresequenz b) codierte Signalpeptid eine Aminosäuresequenz gemäß SEQ ID NO. 4 aufweist. Für derartige Signalpeptide codierende, besonders bevorzugte Nukleinsäuresequenzen sind in SEQ ID NO. 3 angegeben.

Ferner ist es möglich, anstelle der genannten Signalpeptide, die eine Sekretion des Proteins ermöglichen, zu diesen Sequenzen strukturhomologe Sequenzen zu verwenden. Unter einer strukturhomologen Sequenz wird eine Aminosäuresequenz verstanden, deren Aminosäureabfolge eine vergleichbare räumliche Faltung aufweist wie ein Signalpeptid mit der Aminosäuresequenz gemäß SEQ ID NO. 4. Diese räumliche Faltung bewirkt, dass sie von der Wirtszelle als sekretorische Signalsequenz erkannt wird und folglich das die strukturhomologe Signalsequenz enthaltende Protein aus der Wirtszelle ausgeschleust wird. Vorzugsweise erfolgt eine Wechselwirkung mit dem von der Wirtszelle verwendeten Translokationssystem. Die strukturhomologe Aminosäuresequenz bindet daher vorzugsweise unmittelbar oder mittelbar an mindestens eine Komponente des Translokationssystems der Wirtszelle. Unter unmittelbarer Bindung wird eine direkte Interaktion verstanden, unter mittelbarer Bindung wird verstanden, dass die Interaktion über eine oder mehrere weitere Komponenten, insbesondere Proteine oder andere Moleküle, erfolgen kann, die als Adapter fungieren und dementsprechend eine Brückenfunktion haben zwischen der strukturhomologen Aminosäuresequenz und einer Komponente des Translokationssystems der Wirtszelle.

Die Bestimmung der Identität von Nukleinsäure- oder Aminosäuresequenzen erfolgt durch einen Sequenzvergleich. Solch ein Vergleich erfolgt dadurch, dass ähnliche Abfolgen in den Nukleotidsequenzen oder Aminosäuresequenzen einander zugeordnet werden. Dieser Sequenzvergleich erfolgt vorzugsweise basierend auf dem im Stand der Technik etablierten und üblicherweise genutzten BLAST-Algorithmus (vgl. beispielsweise Altschul, S.F., Gish, W., Miller, W., Myers, E.W. & Lipman, D.J. (1990) "Basic local alignment search tool." J. Mol. Biol. 215:403-410, und Altschul, Stephan F.,Thomas L. Madden, Alejandro A. Schaffer, Jinghui Zhang, Hheng Zhang, Webb Miller, and David J. Lipman (1997): "Gapped BLAST and PSI-BLAST: a new generation of protein database search programs"; Nucleic Acids Res., 25, S.3389-3402) und geschieht prinzipiell dadurch, dass ähnliche Abfolgen von Nukleotiden oder Aminosäuren in den Nukleinsäure- bzw. Aminosäuresequenzen einander zugeordnet werden. Eine tabellarische Zuordnung der betreffenden Positionen wird als Alignment bezeichnet. Ein weiterer im Stand der Technik verfügbarer Algorithmus ist der FASTA-Algorithmus. Sequenzvergleiche (Alignments), insbesondere multiple Sequenzvergleiche, werden üblicherweise mit Computerprogrammen erstellt. Häufig genutzt werden beispielsweise die Clustal-Serie (vgl. beispielsweise Chenna et al. (2003): Multiple sequence alignment with the Clustal series of programs. Nucleic Acid Research 31, 3497-3500), T-Coffee (vgl. beispielsweise Notredame et al. (2000): T-Coffee: A novel method for multiple sequence alignments. J. Mol. Biol. 302, 205-217) oder Programme, die auf diesen Programmen bzw. Algorithmen basieren. Im Rahmen der vorliegenden Erfindung werden Sequenzvergleiche und Alignments bevorzugt mit dem Computer-Programm Vector NTI^{®} Suite 10.3 (Invitrogen Corporation, 1600 Faraday Avenue, Carlsbad, Kalifornien, USA) mit den vorgegebenen Standard (Default)-Parametern erstellt.

Solch ein Vergleich erlaubt eine Aussage über die Ähnlichkeit der verglichenen Sequenzen zueinander. Sie wird üblicherweise in Prozent Identität, das heißt dem Anteil der identischen Nukleotide oder Aminosäurereste an denselben bzw. in einem Alignment einander entsprechenden Positionen, angegeben. Der weiter gefasste Begriff der Homologie bezieht bei Aminosäuresequenzen konservierte Aminosäure-Austausche in die Betrachtung mit ein, also Aminosäuren mit ähnlichen Eigenschaften, da diese innerhalb des Proteins meist ähnliche Aktivitäten bzw. Funktionen ausüben. Daher kann die Ähnlichkeit der verglichenen Sequenzen auch Prozent Homologie oder Prozent Ähnlichkeit angegeben sein. Identitäts- und/oder Homologieangaben können über ganze Polypeptide oder Gene oder nur über einzelne Bereiche getroffen werden. Homologe bzw. identische Bereiche von verschiedenen Nukleinsäure- oder Aminosäuresequenzen sind daher durch Übereinstimmungen in den Sequenzen definiert. Sie weisen oftmals gleiche oder ähnliche Funktionen auf. Sie können klein sein und nur wenige Nukleotide bzw. Aminosäuren umfassen. Oftmals üben solche kleinen Bereiche für die Gesamtaktivität des Proteins essentielle Funktionen aus. Es kann daher sinnvoll sein, Sequenzübereinstimmungen nur auf einzelne, gegebenenfalls kleine Bereiche zu beziehen. Soweit nicht anders angegeben beziehen sich Identitäts- bzw. Homologieangaben in der vorliegenden Anmeldung aber auf die Gesamtlänge der jeweils angegebenen Nukleinsäure- oder Aminosäuresäuresequenz.

Das von der Nukleinsäuresequenz b) codierte Protein umfasst ferner eine weitere Aminosäuresequenz. Bei dieser Aminosäuresequenz handelt es sich folglich um die eigentliche Aminosäuresequenz des Proteins ohne Signalpeptid. Vorzugsweise handelt es sich um die Aminosäuresequenz einen reifen Proteins. Unter einem reifen Protein wird dessen fertig prozessierte Form verstanden, da es möglich ist, dass von einem zugehörigen Gen eine immature Form codiert wird, die nach der Translation noch zur reifen Form prozessiert wird. Beispielsweise können immature Formen des Proteins Signal- und/oder Propeptide oder Elongationen am N-Terminus und/oder C-Terminus umfassen, die in der reifen Form nicht mehr vorhanden sind. Beispielsweise umfassen immature Formen von Proteasen, insbesondere Subtilasen und hierunter vor allem Subtilisine, ein Signalpeptid sowie ein Propeptid, die in der reifen (maturen) Form der Protease nicht mehr vorhanden sind. Alternativ handelt es sich bei der weiteren Aminosäuresequenz um die Aminosäuresequenz eines unreifen Proteins, welche ein Propeptid umfasst. Eine derartige Ausgestaltung kommt insbesondere auch für Proteasen, insbesondere Subtilasen und hierunter vor allem Subtilisine in Betracht. In besonders bevorzugten Ausgestaltungen umfasst die weitere Aminosäuresequenz kein weiteres Signalpeptid. Bei derartigen erfindungsgemäßen Ausgestaltungen bewirkt folglich nur das erfindungsgemäße Signalpeptid die Sektretion des Proteins aus einer Wirtszelle.

Die weitere Aminosäuresequenz des Proteins umfasst die Aminosäuresequenz einer Protease, Amylase, Cellulase, Hemicellulase, Mannanase, Tannase, Xylanase, Xanthanase, Xyloglucanase, ß-Glucosidase, einem Pektin-spaltenden Enzym, Carrageenase, Perhydrolase, Oxidase, Oxidoreduktase oder einer Lipase. Ganz besonders bevorzugt umfasst die weitere Aminosäuresequenz des Proteins die Aminosäuresequenz einer Protease und hierunter eines Subtilisins.

Beispielsweise kann eines der nachstehend genannten Enzyme mit einem erfindungsgemäßen Expressionsvektor vorteilhaft hergestellt werden.

Unter den Proteasen sind Subtilisine bevorzugt. Beispiele hierfür sind die Subtilisine BPN' und Carlsberg, die Protease PB92, die Subtilisine 147 und 309, die Alkalische Protease aus Bacillus lentus, Subtilisin DY und die den Subtilasen, nicht mehr jedoch den Subtilisinen im engeren Sinne zuzuordnenden Enzyme Thermitase, Proteinase K und die Proteasen TW3 und TW7. Subtilisin Carlsberg ist in weiterentwickelter Form unter dem Handelsnamen Alcalase^{®} von der Firma Novozymes A/S, Bagsværd, Dänemark, erhältlich. Die Subtilisine 147 und 309 werden unter den Handelsnamen Esperase^{®}, beziehungsweise Savinase^{®} von der Firma Novozymes vertrieben. Von der Protease aus Bacillus lentus DSM 5483 leiten sich die unter der Bezeichnung BLAP^{®} geführten Protease-Varianten ab. Weitere bevorzugte Proteasen sind ferner beispielsweise die unter der Bezeichnung PUR geführten Enzyme. Weitere Proteasen sind ferner die unter den Handelsnamen Durazym^{®}, Relase^{®}, Everlase^{®}, Nafizym^{®}, Natalase^{®}, Kannase^{®} und Ovozyme^{®} von der Firma Novozymes, die unter den Handelsnamen, Purafect^{®}, Purafect^{®} OxP, Purafect^{®} Prime, Excellase^{®} und Properase^{®} von der Firma Genencor, das unter dem Handelsnamen Protosol^{®} von der Firma Advanced Biochemicals Ltd., Thane, Indien, das unter dem Handelsnamen Wuxi^{®} von der Firma Wuxi Snyder Bioproducts Ltd., China, die unter den Handelsnamen Proleather^{®} und Protease P^{®} von der Firma Amano Pharmaceuticals Ltd., Nagoya, Japan, und das unter der Bezeichnung Proteinase K-16 von der Firma Kao Corp., Tokyo, Japan, erhältlichen Enzyme. Bevorzugt sind ferner auch die Proteasen aus Bacillus gibsonii und Bacillus pumilus, die offenbart sind in den internationalen Patentanmeldungen WO2008/086916 und WO2007/131656.

Beispiele für Amylasen sind die α-Amylasen aus Bacillus licheniformis, aus Bacillus amyloliquefaciens oder aus Bacillus stearothermophilus sowie insbesondere auch deren für den Einsatz in Wasch- oder Reinigungsmitteln verbesserte Weiterentwicklungen. Das Enzym aus Bacillus licheniformis ist von dem Unternehmen Novozymes unter dem Namen Termamyl^{®} und von dem Unternehmen Danisco/Genencor unter dem Namen Purastar^{®}ST erhältlich. Weiterentwicklungsprodukte dieser α-Amylase sind von dem Unternehmen Novozymes unter den Handelsnamen Duramyl^{®} und Termamyl^{®}ultra, von dem Unternehmen Danisco/Genencor unter dem Namen Purastar^{®}OxAm und von dem Unternehmen Daiwa Seiko Inc., Tokyo, Japan, als Keistase^{®} erhältlich. Die α-Amylase von Bacillus amyloliquefaciens wird von dem Unternehmen Novozymes unter dem Namen BAN^{®} vertrieben, und abgeleitete Varianten von der α-Amylase aus Bacillus stearothermophilus unter den Namen BSG^{®} und Novamyl^{®}, ebenfalls von dem Unternehmen Novozymes. Des Weiteren sind die α-Amylase aus Bacillus sp. A 7-7 (DSM 12368) und die Cyclodextrin-Glucanotransferase (CGTase) aus Bacillus agaradherens (DSM 9948) zu nennen. Ebenso sind Fusionsprodukte aller genannten Moleküle einsetzbar. Darüber hinaus sind die unter den Handelsnamen Fungamyl^{®} von dem Unternehmen Novozymes erhältlichen Weiterentwicklungen der α-Amylase aus Aspergillus niger und A. oryzae geeignet. Weitere vorteilhafte Handelsprodukte sind beispielsweise die Amylase Powerase^{®} von dem Unternehmen Danisco/Genencor und die Amylasen Amylase-LT^{®}, Stainzyme^{®} und Stainzyme plus^{®}, letztere von dem Unternehmen Novozymes. Auch durch Punktmutationen erhältliche Varianten dieser Enzyme können erfindungsgemäß hergestellt werden. Weitere bevorzugte Amylasen sind offenbart in den internationalen Offenlegungsschriften WO 00/60060, WO 03/002711, WO 03/054177 und WO07/079938, auf deren Offenbarung daher ausdrücklich verwiesen wird bzw. deren diesbezüglicher Offenbarungsgehalt daher ausdrücklich in die vorliegende Patentanmeldung mit einbezogen wird. Erfindungsgemäß herzustellende Amylasen sind ferner vorzugsweise α-Amylasen.

Beispiele für Lipasen oder Cutinasen sind die ursprünglich aus Humicola lanuginosa (Thermomyces lanuginosus) erhältlichen, beziehungsweise weiterentwickelten Lipasen, insbesondere solche mit dem Aminosäureaustausch D96L. Sie werden beispielsweise von der Firma Novozymes unter den Handelsnamen Lipolase^{®}, Lipolase^{®}Ultra, LipoPrime^{®}, Lipozyme^{®} und Lipex^{®} vertrieben. Des Weiteren sind beispielsweise die Cutinasen herstellbar, die ursprünglich aus Fusarium solani pisi und Humicola insolens isoliert worden sind. Von der Firma Danisco/Genencor sind beispielsweise die Lipasen beziehungsweise Cutinasen herstellbar, deren Ausgangsenzyme ursprünglich aus Pseudomonas mendocina und Fusarium solanii isoliert worden sind. Als weitere wichtige Handelsprodukte sind die ursprünglich von der Firma Gist-Brocades (inzwischen Danisco/Genencor) vertriebenen Präparationen M1 Lipase^{®} und Lipomax^{®} und die von der Firma Meito Sangyo KK, Japan, unter den Namen Lipase MY-30^{®}, Lipase OF^{®} und Lipase PL^{®} vertriebenen Enzyme zu erwähnen, ferner das Produkt Lumafast^{®} von der Firma Danisco/Genencor.

Beispiele für Cellulasen (Endoglucanasen, EG) umfassen Sequenzen der pilzlichen, Endoglucanase(EG)-reichen Cellulase-Präparation beziehungsweise deren Weiterentwicklungen, die von dem Unternehmen Novozymes unter dem Handelsnamen Celluzyme^{®} angeboten wird. Die ebenfalls von dem Unternehmen Novozymes erhältlichen Produkte Endolase^{®} und Carezyme^{®} basieren auf der 50 kD-EG, beziehungsweise der 43 kD-EG aus Humicola insolens DSM 1800. Weitere herstellbare Handelsprodukte dieses Unternehmens sind Cellusoft^{®}, Renozyme^{®} und Celluclean^{®}. Weiterhin herstellbar sind beispielsweise Cellulasen, die von dem Unternehmen AB Enzymes, Finnland, unter den Handelsnamen Ecostone^{®} und Biotouch^{®} erhältlich sind, und die zumindest zum Teil auf der 20 kD-EG aus Melanocarpus basieren. Weitere Cellulasen von dem Unternehmen AB Enzymes sind Econase^{®} und Ecopulp^{®}. Weitere geeignete Cellulasen sind aus Bacillus sp. CBS 670.93 und CBS 669.93, wobei die aus Bacillus sp. CBS 670.93 von dem Unternehmen Danisco/Genencor unter dem Handelsnamen Puradax^{®} erhältlich ist. Weitere herstellbare Handelsprodukte des Unternehmens Danisco/Genencor sind "Genencor detergent cellulase L" und IndiAge^{®}Neutra.

Auch durch Punktmutationen erhältliche Varianten dieser Enzyme können erfindungsgemäß hergestellt werden. Besonders bevorzugte Cellulasen sind Thielavia terrestris Cellulasevarianten, die in der internationalen Offenlegungsschrift WO 98/12307 offenbart sind, Cellulasen aus Melanocarpus, insbesondere Melanocarpus albomyces, die in der internationalen Offenlegungsschrift WO 97/14804 offenbart sind, Cellulasen vom EGIII-Typ aus Trichoderma reesei, die in der europäischen Patentanmeldung EP 1 305 432 offenbart sind bzw. hieraus erhältliche Varianten, insbesondere diejenigen, die offenbart sind in den europäischen Patentanmeldungen EP 1240525 und EP 1305432, sowie Cellulasen, die offenbart sind in den internationalen Offenlegungsschriften WO 1992006165, WO 96/29397 und WO 02/099091. Auf deren jeweilige Offenbarung wird daher ausdrücklich verwiesen bzw. deren diesbezüglicher Offenbarungsgehalt wird daher ausdrücklich in die vorliegende Patentanmeldung mit einbezogen.

Ferner können weitere Enzyme hergestellt werden, die unter dem Begriff Hemicellulasen zusammengefasst werden. Hierzu gehören beispielsweise Mannanasen, Xanthanlyasen, Xanthanasen, Xyloglucanasen, Xylanasen, Pullulanasen, Pektin-spaltende Enzyme und ß-Glucanasen. Die aus Bacillus subtilis gewonnene ß-Glucanase ist unter dem Namen Cereflo^{®} von der Firma Novozymes erhältlich. Erfindungsgemäß besonders bevorzugte Hemicellulasen sind Mannanasen, welche beispielsweise unter den Handelsnamen Mannaway^{®} von dem Unternehmen Novozymes oder Purabrite^{®} von dem Unternehmen Genencor vertrieben werden. Zu den Pektin-spaltenden Enzymen werden im Rahmen der vorliegenden Erfindung ebenfalls Enzyme gezählt mit den Bezeichnungen Pektinase, Pektatlyase, Pektinesterase, Pektindemethoxylase, Pektinmethoxylase, Pektinmethylesterase, Pektase, Pektinmethylesterase, Pektinoesterase, Pektinpektylhydrolase, Pektindepolymerase, Endopolygalacturonase, Pektolase, Pektinhydrolase, Pektin-Polygalacturonase, Endo-Polygalacturonase, Poly-α-1,4-Galacturonid Glycanohydrolase, Endogalacturonase, Endo-D-galacturonase, Galacturan 1,4-α-Galacturonidase, Exopolygalacturonase, Poly(galacturonat) Hydrolase, Exo-D-Galacturonase, Exo-D-Galacturonanase, Exopoly-D-Galacturonase, Exo-poly-α-Galacturonosidase, Exopolygalacturonosidase oder Exopolygalacturanosidase. Beispiele für diesbezüglich geeignete Enzyme sind beispielsweise unter den Namen Gamanase^{®}, PektinexAR^{®}, X-Pect^{®} oder Pectaway^{®} von dem Unternehmen Novozymes, unter dem Namen Rohapect UF^{®}, Rohapect TPL^{®}, Rohapect PTE100^{®}, Rohapect MPE^{®}, Rohapect MA plus HC, Rohapect DA12L^{®}, Rohapect 10L^{®}, Rohapect B1L^{®} von dem Unternehmen AB Enzymes und unter dem Namen Pyrolase^{®} von dem Unternehmen Diversa Corp., San Diego, CA, USA erhältlich.

Ferner können auch Oxidoreduktasen, beispielsweise Oxidasen, Oxygenasen, Katalasen, Peroxidasen, wie Halo-, Chloro-, Bromo-, Lignin-, Glucose- oder Manganperoxidasen, Dioxygenasen oder Laccasen (Phenoloxidasen, Polyphenoloxidasen) hergestellt werden. Als geeignete Handelsprodukte sind Denilite^{®} 1 und 2 der Firma Novozymes zu nennen. Weitere Enzyme sind in den internationalen Patentanmeldungen WO 98/45398, WO 2005/056782, WO 2004/058961 sowie WO 2005/124012 offenbart.

In einer weiteren Ausführungsform der Erfindung ist die weitere Aminosäuresequenz nicht natürlicherweise zusammen mit dem Signalpeptid in einer Polypeptidkette in einem Mikroorganismus vorhanden. Folglich handelt es sich bei dem von der Nukleinsäuresequenz b) codierten Protein um ein rekombinantes Protein. Nicht natürlicherweise vorhanden bedeutet daher, dass die beiden Aminosäuresequenzen keine Bestandteile eines eigenen Proteins des Mikroorganismus sind. Ein Protein, welches das Signalpeptid und die weitere Aminosäuresequenz umfasst, kann folglich in dem Mikroorganismus nicht von einer Nukleinsäuresequenz exprimiert werden, die Teil der chromosomalen DNA des Mikroorganismus in seiner Wildtyp-Form ist. Ein derartiges Protein und/oder die hierfür jeweils codierende Nukleinsäuresequenz ist folglich in der Wildtyp-Form des Mikroorganismus nicht vorhanden und/oder kann aus der Wildtyp-Form des Mikroorganismus nicht isoliert werden. Beide Sequenzen - Signalpeptid und weitere Aminosäuresequenz - müssen in einer Wildtyp-Form eines Mikroorganismus daher zwei unterschiedlichen Polypeptidketten zugeordnet sein, sofern sie überhaupt beide in der Wildtyp-Form eines Mikroorganismus vorhanden sind bzw. sein können. Im Rahmen dieser Ausgestaltung der Erfindung wurden daher Signalpeptid und weitere Aminosäuresequenz bzw. die für sie codierenden Nukleinsäuren mit Hilfe gentechnischer Verfahren neu kombiniert, und diese Kombination von Signalpeptid und weiterer Aminosäuresequenz existiert in der Natur nicht. In der Wildtyp-Form eines Mikroorganismus ist eine derartige Verknüpfung von dem Signalpeptid mit der weiteren Aminosäuresequenz folglich nicht vorhanden, und zwar weder auf DNA- noch auf Proteinebene. Das Signalpeptid wie auch die weitere Aminosäuresequenz bzw. die hierfür jeweils codierenden Nukleinsäuresequenzen können aber jeweils natürlichen Ursprungs sein, jedoch existiert deren Kombination in der Natur nicht. Signalpeptid und weitere Aminosäuresequenz selbst können aber aus dem gleichen Mikroorganismus oder auch aus verschiedenen Mikroorganismen stammen.

In einer bevorzugten Ausführungsform ist eine erfindungsgemäße Nukleinsäure dadurch gekennzeichnet, dass sie eine nicht natürliche Nukleinsäure ist. Nicht natürlich bedeutet, dass eine erfindungsgemäße Nukleinsäure nicht aus einem in der Natur vorkommenden Organismus in seiner Wildtyp-Form isoliert werden kann. Insbesondere und bezogen auf Wildtyp-Bakterien ist eine erfindungsgemäße Nukleinsäure daher keine bakterieneigene Nukleinsäure.

Bevorzugt stammen die Sequenzen (a) und (b) nicht von demselben bzw. denselben Organsimen, insbesondere Bakterien, sondern stammen von unterschiedlichen Organismen, insbesondere Bakterien. Unterschiedliche Bakterien sind beispielsweise Bakterien, die unterschiedlichen Stämmen oder Arten oder Gattungen angehören.

In einer weiteren Ausführungsform der Erfindung ist der Expressionsvektor dadurch gekennzeichnet, dass das Signalpeptid N-terminal von der weiteren Aminosäuresequenz in dem von der Nukleinsäuresequenz b) codierten Protein angeordnet ist. Das von der Nukleinsäuresequenz b) codierte Protein weist daher folgende Struktur auf: N-Terminus - Signalpeptid - (optionale zusätzliche Aminosäuresequenz) - weitere Aminosäuresequenz - C-Terminus. Eine derartige Struktur des zu exprimierenden Proteins hat sich als besonders vorteilhaft herausgestellt.

In einer weiteren Ausführungsform der Erfindung ist der Expressionsvektor dadurch gekennzeichnet, dass das von der Nukleinsäuresequenz b) codierte Protein ferner eine Verbindungssequenz umfasst, die zwischen dem Signalpeptid und der weiteren Aminosäuresequenz des Proteins angeordnet ist. Das von der Nukleinsäuresequenz b) codierte Protein weist daher folgende Struktur auf: N-Terminus - Signalpeptid - Verbindungssequenz (auch "Kupplung" oder "Spacer") - weitere Aminosäuresequenz - C-Terminus. Eine derartige Struktur des zu exprimierenden Proteins hat sich ebenfalls als besonders vorteilhaft herausgestellt. Vorzugsweise weist die Verbindungssequenz eine Länge zwischen 1 und 50 Aminosäuren, zwischen 2 und 25 Aminosäuren, zwischen 2 und 15 Aminosäuren, zwischen 3 und 10 Aminosäuren und besonders bevorzugt zwischen 3 und 5 Aminosäuren auf. Ein Beispiel für eine besonders bevorzugte Verbindungssequenz ist die Aminosäureabfolge Alanin, Glutaminsäure und Phenylalanin (vom N- zum C-Terminus).

In einer weiteren Ausführungsform der Erfindung ist der Expressionsvektor dadurch gekennzeichnet, dass die weitere Aminosäuresequenz des Proteins die Aminosäuresequenz einer Protease umfasst, wobei die Aminosäuresequenz der Protease mindestens zu 80% identisch zu SEQ ID NO. 7 ist. Bevorzugt ist die Aminosäuresequenz der Protease mindestens zu 81%, 82%, 83%, 84%, 85%, 86%, 87%, 88%, 89%, 90%, 91%, 92%, 93%, 94%, 95%, 96%, 97%, 98%, 99% und ganz besonders bevorzugt zu 100% identisch zu SEQ ID NO. 7.

Alternativ umfasst die weitere Aminosäuresequenz des Proteins die Aminosäuresequenz einer Protease, die mindestens zu 80% identisch zu SEQ ID NO. 8 ist. Bevorzugt ist die Aminosäuresequenz der Protease zu mindestens 81%, 82%, 83%, 84%, 85%, 86%, 87%, 88%, 89%, 90%, 91%, 92%, 93%, 94%, 95%, 96%, 97%, 98%, 99% und ganz besonders bevorzugt zu 100% identisch zu SEQ ID NO. 8.

Alternativ umfasst die weitere Aminosäuresequenz des Proteins die Aminosäuresequenz einer Protease, die mindestens zu 80% identisch zu SEQ ID NO. 9 ist. Bevorzugt ist die Aminosäuresequenz der Protease zu mindestens 81%, 82%, 83%, 84%, 85%, 86%, 87%, 88%, 89%, 90%, 91%, 92%, 93%, 94%, 95%, 96%, 97%, 98%, 99% und ganz besonders bevorzugt zu 100% identisch zu SEQ ID NO. 9.

Alternativ umfasst die weitere Aminosäuresequenz des Proteins die Aminosäuresequenz einer Protease, die mindestens zu 80% identisch zu SEQ ID NO. 10 ist und an Position 99 in der Zählung gemäß SEQ ID NO. 10 die Aminosäure Glutaminsäure (E) oder Asparaginsäure (D) aufweist. Bevorzugt ist die Aminosäuresequenz der Protease zu mindestens 81%, 82%, 83%, 84%, 85%, 86%, 87%, 88%, 89%, 90%, 91%, 92%, 93%, 94%, 95%, 96%, 97%, 98%, 99% und ganz besonders bevorzugt identisch zu SEQ ID NO. 10 in den Positionen 1 bis 98 und 100 bis 269 in der Zählung gemäß SEQ ID NO. 10.

Alternativ umfasst die weitere Aminosäuresequenz des Proteins die Aminosäuresequenz einer Protease, die mindestens zu 80% identisch zu SEQ ID NO. 10 ist und an Position 99 in der Zählung gemäß SEQ ID NO. 10 die Aminosäure Glutaminsäure (E) oder Asparaginsäure (D) aufweist und ferner in der Zählung gemäß SEQ ID NO. 10 mindestens eine der folgenden Aminosäuren aufweist:
(a) Threonin an Position 3 (3T),
(b) Isoleucin an Position 4 (4I),
(c) Alanin, Threonin oderArginin an Position 61 (61A, 61T oder 61R),
(d) Asparaginsäure oder Glutaminsäure an Position 154 (154D oder 154E),
(e) Prolin an Position 188 (188P),
(f) Methionin an Position 193 (193M),
(g) Isoleucin an Position 199 (199I),
(h) Asparaginsäure, Glutaminsäure oder Glycin an Position 211 (211D, 211E oder 211G),
(i) Kombinationen der Aminosäuren (a) bis (h).

Bevorzugt ist die Aminosäuresequenz dieser Protease zu mindestens 81%, 82%, 83%, 84%, 85%, 86%, 87%, 88%, 89%, 90%, 91%, 92%, 93%, 94%, 95%, 96%, 97%, 98%, 99% und ganz besonders bevorzugt identisch zu SEQ ID NO. 10 in allen nicht veränderten oder nicht für eine Veränderung vorgesehenen Positionen. Ganz besonders bevorzugt umfasst die weitere Aminosäuresequenz des Proteins daher die Aminosäuresequenz einer Protease, die eine gegenüber SEQ ID NO. 10 in mindestens zwei Positionen veränderte Aminosäuresequenz aufweist, wobei die erste Veränderung in der Zählung gemäß SEQ ID NO. 10 Glutaminsäure an Position 99 ist und die zweite Veränderung in der Zählung gemäß SEQ ID NO. 10 ausgewählt ist aus der Gruppe bestehend aus:
(a) Threonin an Position 3 (3T),
(b) Isoleucin an Position 4 (4I),
(c) Alanin, Threonin oder Arginin an Position 61 (61A, 61T oder 61R),
(d) Asparaginsäure oder Glutaminsäure an Position 154 (154D oder 154E),
(e) Prolin an Position 188 (188P),
(f) Methionin an Position 193 (193M),
(g) Isoleucin an Position 199 (199I),
(h) Asparaginsäure, Glutaminsäure oder Glycin an Position 211 (211D, 211E oder 211G),
(i) Kombinationen der Aminosäuren (a) bis (h).

Ebenfalls ganz besonders bevorzugt umfasst die weitere Aminosäuresequenz des Proteins die Aminosäuresequenz einer Protease, die eine gegenüber SEQ ID NO. 10 in mindestens zwei Positionen veränderte Aminosäuresequenz aufweist, wobei die erste Veränderung in der Zählung gemäß SEQ ID NO. 10 Asparaginsäure an Position 99 ist und die zweite Veränderung in der Zählung gemäß SEQ ID NO. 10 ausgewählt ist aus der Gruppe bestehend aus:
(a) Threonin an Position 3 (3T),
(b) Isoleucin an Position 4 (4I),
(c) Alanin, Threonin oder Arginin an Position 61 (61A, 61T oder 61R),
(d) Asparaginsäure oder Glutaminsäure an Position 154 (154D oder 154E),
(e) Prolin an Position 188 (188P),
(f) Methionin an Position 193 (193M),
(g) Isoleucin an Position 199 (199I),
(h) Asparaginsäure, Glutaminsäure oder Glycin an Position 211 (211D, 211E oder 211G),
(i) Kombinationen der Aminosäuren (a) bis (h).

Es wurde festgestellt, dass auch die vorstehend genannten Proteasen mit erfindungsgemäßen Expressionsvektoren besonders vorteilhaft hergestellt werden können. Für derartige Ausführungsformen der Erfindung hat sich herausgestellt, dass sich mit solchen Kombinationen von Signalpeptiden und Subtilisinen besonders gute Produktausbeuten in einer Fermentation erzielen lassen. Angegeben sind diesbezüglich die Aminosäuresequenzen der reifen Proteasen, also der fertig prozessierten Produkte. In einem erfindungsgemäßen Expressionsvektor können diesbezüglich auch weitere Sequenzen der immaturen Protease, insbesondere beispielsweise Propeptide, mit umfasst sein. In einem solchen Fall umfasst die weitere Aminosäuresequenz des Proteins die Aminosäuresequenz der Protease und des Propeptids. Eine weitere Ausgestaltung der Erfindung ist folglich dadurch gekennzeichnet, dass die weitere Aminosäuresequenz des Proteins die Aminosäuresequenz einer Protease, insbesondere einer Protease wie vorstehend beschrieben, nebst einem bzw. ihrem Propeptid umfasst.

Generell gilt, dass die weitere Aminosäuresequenz des Proteins nicht lediglich die Aminosäuresequenz eines reifen Proteins umfassen muss; vielmehr können weitere Aminosäuresequenzen wie beispielsweise Propeptide von dieser Aminosäuresequenz mit umfasst sein. Dies gilt nicht nur für Proteasen, sondern für alle Proteine, insbesondere auch alle anderen Arten von Enzymen.

Erfindungsgemäße Nukleinsäuren und Expressionsvektoren können über an sich bekannte Verfahren zur Veränderung von Nukleinsäuren erzeugt werden. Solche sind beispielsweise in einschlägigen Handbüchern wie dem von Fritsch, Sambrook und Maniatis, "Molecular cloning: a laboratory manual", Cold Spring Harbour Laboratory Press, New York, 1989, dargestellt und dem Fachmann auf dem Gebiet der Biotechnologie geläufig. Beispiele für solche Verfahren sind die chemische Synthese oder die Polymerase-Kettenreaktion (PCR), optional in Verbindung mit weiteren molekularbiologischen und/oder chemischen bzw. biochemischen Standardmethoden.

Mit allen genannten Erfindungsgegenständen und Ausführungsformen sind als weitere Erfindungsgegenstände nicht menschliche Wirtszellen, die erfindungsgemäße Vektoren beinhalten, Herstellungsverfahren, in denen entsprechende Wirtszellen eingesetzt werden sowie Verwendungen entsprechender Vektoren oder Wirtszellen verbunden. Daher betreffen die vorstehenden Ausführungen diese Erfindungsgegenstände entsprechend.

Ein weiterer Gegenstand der Erfindung ist eine nicht menschliche Wirtszelle, die einen erfindungsgemäßen Expressionsvektor beinhaltet. Ein erfindungsgemäßer Expressionsvektor wird bevorzugt in die Wirtszelle eingebracht durch deren Transformation. Erfindungsgemäß bevorzugt erfolgt dieses dadurch, dass in einen Mikroorganismus ein erfindungsgemäßer Vektor transformiert wird, der dann eine erfindungsgemäße Wirtszelle darstellt. Alternativ können auch einzelne Komponenten, d.h. Nukleinsäure-Teile bzw. -Fragmente, beispielsweise die Komponenten (a) und/oder (b) eines erfindungsgemäßen Vektors, derart in eine Wirtszelle eingebracht werden, dass die dann resultierende Wirtszelle einen erfindungsgemäßen Vektor enthält. Dieses Vorgehen eignet sich besonders dann, wenn die Wirtszelle bereits einen oder mehrere Bestandteile eines erfindungsgemäßen Vektors enthält und die weiteren Bestandteile dann entsprechend ergänzt werden. Verfahren zur Transformation von Zellen sind im Stand der Technik etabliert und dem Fachmann hinlänglich bekannt. Als Wirtszellen eignen sich prinzipiell alle Zellen, das heißt prokaryotische oder eukaryotische Zellen. Bevorzugt sind solche Wirtszellen, die sich genetisch vorteilhaft handhaben lassen, was beispielsweise die Transformation mit dem Vektor und dessen stabile Etablierung angeht, beispielsweise einzellige Pilze oder Bakterien. Ferner zeichnen sich bevorzugte Wirtszellen durch eine gute mikrobiologische und biotechnologische Handhabbarkeit aus. Das betrifft beispielsweise leichte Kultivierbarkeit, hohe Wachstumsraten, geringe Anforderungen an Fermentationsmedien und gute Produktions- und Sekretionsraten für Fremdproteine. Häufig müssen aus der Fülle an verschiedenen im Stand der Technik zur Verfügung stehenden Systemen die optimalen Expressionssysteme für den Einzelfall experimentell ermittelt werden.

Weitere bevorzugte Ausführungsformen stellen solche Wirtszellen dar, die aufgrund genetischer Regulationselemente, die beispielsweise auf dem Vektor zur Verfügung gestellt werden, aber auch von vornherein in diesen Zellen vorhanden sein können, in ihrer Aktivität regulierbar sind. Beispielsweise durch kontrollierte Zugabe von chemischen Verbindungen, die als Aktivatoren dienen, durch Änderung der Kultivierungsbedingungen oder bei Erreichen einer bestimmten Zelldichte können diese zur Expression angeregt werden. Dies ermöglicht eine wirtschaftliche Produktion der Proteine.

Bevorzugte Wirtszellen sind prokaryontische oder bakterielle Zellen. Bakterien zeichnen sich durch kurze Generationszeiten und geringe Ansprüche an die Kultivierungsbedingungen aus. Dadurch können kostengünstige Verfahren etabliert werden. Zudem verfügt der Fachmann bei Bakterien in der Fermentationstechnik über einen reichhaltigen Erfahrungsschatz. Für eine spezielle Produktion können aus verschiedensten, im Einzelfall experimentell zu ermittelnden Gründen wie Nährstoffquellen, Produktbildungsrate, Zeitbedarf usw., gramnegative oder grampositive Bakterien geeignet sein.

Bei gramnegativen Bakterien wie beispielsweise Escherichia coli wird eine Vielzahl von Polypeptiden in den periplasmatischen Raum sezerniert, also in das Kompartiment zwischen den beiden die Zellen einschließenden Membranen. Dies kann für spezielle Anwendungen vorteilhaft sein. Ferner können auch gramnegative Bakterien so ausgestaltet werden, dass sie die exprimierten Polypeptide nicht nur in den periplasmatischen Raum, sondern in das das Bakterium umgebende Medium ausschleusen. Grampositive Bakterien wie beispielsweise Bacilli oder Actinomyceten oder andere Vertreter der Actinomycetales besitzen demgegenüber keine äußere Membran, so dass sezernierte Proteine sogleich in das die Bakterien umgebende Medium, in der Regel das Nährmedium, abgegeben werden, aus welchem sich die exprimierten Polypeptide aufreinigen lassen. Sie können aus dem Medium direkt isoliert oder weiter prozessiert werden. Zudem sind grampositive Bakterien mit den meisten Herkunftsorganismen für technisch wichtige Enzyme verwandt oder identisch und bilden meist selbst vergleichbare Enzyme, so dass sie über eine ähnliche Codon-Usage verfügen und ihr Protein-Syntheseapparat naturgemäß entsprechend ausgerichtet ist.

Unter Codon-Usage wird die Übersetzung des genetischen Codes in Aminosäuren verstanden, d.h. welche Nukleotidfolge (Triplett oder Basentriplett) für welche Aminosäure bzw. für welche Funktion, beispielsweise Beginn und Ende des zu translatierenden Bereichs, Bindungsstellen für verschiedene Proteine, usw., kodiert. So besitzt jeder Organismus, insbesondere jeder Produktionsstamm eine bestimmte Codon-Usage. Es kann zu Engpässen in der Proteinbiosynthese kommen, wenn die auf der transgenen Nukleinsäure liegenden Codons in der Wirtszelle einer vergleichsweise geringen Zahl von beladenen tRNAs gegenüberstehen. Synonyme Codons codieren dagegen für dieselben Aminosäuren und können in Abhängigkeit vom Wirt besser translatiert werden. Dieses gegebenenfalls notwendige Umschreiben hängt somit von der Wahl des Expressionssystems ab. Insbesondere bei Proben aus unbekannten, eventuell nicht kultivierbaren Organismen kann eine entsprechende Anpassung notwendig sein.

Die vorliegende Erfindung ist prinzipiell auf alle Mikroorganismen, insbesondere auf alle fermentierbaren Mikroorganismen, besonders bevorzugt auf solche der Gattung Bacillus, anwendbar und führt dazu, dass sich durch den Einsatz solcher Mikroorganismen als Produktionsorganismen eine erhöhte Produktausbeute in einer Fermentation verwirklichen lässt. Solche Mikroorganismen stellen bevorzugte Wirtszellen im Sinne der Erfindung dar.

In einer weiteren Ausführungsform der Erfindung ist die Wirtszelle daher dadurch gekennzeichnet, dass sie ein Bakterium ist, bevorzugt eines, das ausgewählt ist aus der Gruppe der Gattungen von Escherichia, Klebsiella, Bacillus, Staphylococcus, Corynebakterium, Arthrobacter, Streptomyces, Stenotrophomonas und Pseudomonas, weiter bevorzugt eines, das ausgewählt ist aus der Gruppe von Escherichia coli, Klebsiella planticola, Bacillus licheniformis, Bacillus lentus, Bacillus amyloliquefaciens, Bacillus subtilis, Bacillus alcalophilus, Bacillus globigii, Bacillus gibsonii, Bacillus clausii, Bacillus halodurans, Bacillus pumilus, Staphylococcus carnosus, Corynebacterium glutamicum, Arthrobacter oxidans, Streptomyces lividans, Streptomyces coelicolor und Stenotrophomonas maltophilia. Ganz besonders bevorzugt ist Bacillus licheniformis.

Die Wirtszelle kann aber auch eine eukaryontische Zelle sein, die dadurch gekennzeichnet ist, dass sie einen Zellkern besitzt. Einen weiteren Gegenstand der Erfindung stellt daher eine Wirtszelle dar, die dadurch gekennzeichnet ist, dass sie einen Zellkern besitzt.

Im Gegensatz zu prokaryontischen Zellen sind eukaryontische Zellen in der Lage, das gebildete Protein posttranslational zu modifizieren. Beispiele dafür sind Pilze wie Actinomyceten oder Hefen wie Saccharomyces oder Kluyveromyces. Dies kann beispielsweise dann besonders vorteilhaft sein, wenn die Proteine im Zusammenhang mit ihrer Synthese spezifische Modifikationen erfahren sollen, die derartige Systeme ermöglichen. Zu den Modifikationen, die eukaryontische Systeme besonders im Zusammenhang mit der Proteinsynthese durchführen, gehören beispielsweise die Bindung niedermolekularer Verbindungen wie Membrananker oder Oligosaccharide. Derartige Oligosaccharid-Modifikationen können beispielsweise zur Senkung der Allergenizität eines exprimierten Proteins wünschenswert sein. Auch eine Coexpression mit den natürlicherweise von derartigen Zellen gebildeten Enzymen, wie beispielsweise Cellulasen, kann vorteilhaft sein. Ferner können sich beispielsweise thermophile pilzliche Expressionssysteme besonders zur Expression temperaturbeständiger Varianten eignen.

Als Produkte, die während der Fermentation gebildet werden, werden im Rahmen der Erfindung Proteine betrachtet, die von der Nukleinsäuresequenz (b) codiert werden, insbesondere solche, wie sie vorstehend beschrieben sind. Hierbei handelt es sich um Proteasen, Amylasen, Cellulasen, Hemicellulasen, Mannanasen, Tannasen, Xylanasen, Xanthanasen, Xyloglucanasen, ß-Glucosidasen, Pektin-spaltendes Enzyme, Carrageenasen, Perhydrolasen, Oxidasen, Oxidoreduktasen oder Lipasen, besonders bevorzugt um Proteasen und ganz besonders bevorzugt um Subtilisine.

Die Wirtszellen können ferner hinsichtlich ihrer Anforderungen an die Kulturbedingungen verändert sein, andere oder zusätzliche Selektionsmarker aufweisen oder andere oder zusätzliche Proteine exprimieren. Es kann sich insbesondere um solche Wirtszellen handeln, die mehrere Proteine oder Enzyme exprimieren. Bevorzugt sezernieren sie diese in das die Wirtszellen umgebende Medium.

Die erfindungsgemäßen Wirtszellen werden in an sich bekannter Weise kultiviert und fermentiert, beispielsweise in diskontinuierlichen oder kontinuierlichen Systemen. Im ersten Fall wird ein geeignetes Nährmedium mit den Wirtszellen beimpft und das Produkt nach einem experimentell zu ermittelnden Zeitraum aus dem Medium geerntet. Kontinuierliche Fermentationen zeichnen sich durch Erreichen eines Fließgleichgewichts aus, in dem über einen vergleichsweise langen Zeitraum Zellen teilweise absterben aber auch nachwachsen und gleichzeitig Produkt aus dem Medium entnommen werden kann.

Erfindungsgemäße Wirtszellen werden bevorzugt verwendet, um Proteine herzustellen, die von der Nukleinsäuresequenz (b) codiert werden. Ein weiterer Gegenstand der Erfindung ist daher ein Verfahren zur Herstellung eines Proteins umfassend
a) Kultivieren einer erfindungsgemäßen Wirtszelle
b) Isolieren des Proteins aus dem Kulturmedium oder aus der Wirtszelle, dadurch gekennzeichnet, dass das Protein eine Protease, Amylase, Cellulase, Hemicellulase, Mannanase, Tannase, Xylanase, Xanthanase, Xyloglucanase, ß-Glucosidase, Pektin-spaltendes Enzym, Carrageenase, Perhydrolase, Oxidase, Oxidoreduktase oder eine Lipase ist.

Dieser Erfindungsgegenstand umfasst bevorzugt Fermentationsverfahren. Fermentationsverfahren sind an sich aus dem Stand der Technik bekannt und stellen den eigentlichen großtechnischen Produktionsschritt dar, in der Regel gefolgt von einer geeigneten Aufreinigungsmethode des hergestellten Produktes, beispielsweise des Proteins. Alle Fermentationsverfahren, die auf einem entsprechenden Verfahren zur Herstellung einer Protease, Amylase, Cellulase, Hemicellulase, Mannanase, Tannase, Xylanase, Xanthanase, Xyloglucanase, ß-Glucosidase, Pektin-spaltendes Enzym, Carrageenase, Perhydrolase, Oxidase, Oxidoreduktase oder einer Lipase beruhen, stellen Ausführungsformen dieses Erfindungsgegenstandes dar.

Hierbei müssen die jeweils optimalen Bedingungen für die Herstellungsverfahren, insbesondere die optimalen Kulturbedingungen für die benutzten Wirtszellen, nach dem Wissen des Fachmanns experimentell ermittelt werden, beispielsweise hinsichtlich Fermentationsvolumen und/oder Medienzusammensetzung und/oder Sauerstoffversorgung und/oder Rührergeschwindigkeit.

Fermentationsverfahren, die dadurch gekennzeichnet sind, dass die Fermentation über eine Zulaufstrategie durchgeführt wird, kommen insbesondere in Betracht. Hierbei werden die Medienbestandteile, die durch die fortlaufende Kultivierung verbraucht werden, zugefüttert; man spricht auch von einer Zufütterungsstrategie. Hierdurch können beträchtliche Steigerungen sowohl in der Zelldichte als auch in der Zellmasse bzw. Trockenmasse und/oder vor allem der Aktivität des interessierenden Proteins, vorzugsweise Enzyms, erreicht werden.

Ferner kann die Fermentation auch so gestaltet werden, dass unerwünschte Stoffwechselprodukte herausgefiltert oder durch Zugabe von Puffer oder jeweils passende Gegenionen neutralisiert werden.

Das hergestellte Protein kann aus dem Fermentationsmedium geerntet werden. Ein solches Fermentationsverfahren ist gegenüber einer Isolation des Polypeptids aus der Wirtszelle, d.h. einer Produktaufbereitung aus der Zellmasse (Trockenmasse) vorteilhaft. Erfindungsgemäß werden mit den Signalpeptiden diesbezüglich geeignete Sekretionsmarker zur Verfügung gestellt.

Alle vorstehend ausgeführten Sachverhalte können zu Verfahren kombiniert werden, um Proteine herzustellen. Es ist diesbezüglich eine Vielzahl von Kombinationsmöglichkeiten von Verfahrensschritten denkbar. Das optimale Verfahren muss für jeden konkreten Einzelfall ermittelt werden.

Ein weiterer Gegenstand der Erfindung ist die Verwendung eines erfindungsgemäßen Expressionsvektors oder einer erfindungsgemäßen Wirtszelle zur Herstellung einer Protease, Amylase, Cellulase, Hemicellulase, Mannanase, Tannase, Xylanase, Xanthanase, Xyloglucanase, ß-Glucosidase, Pektin-spaltendes Enzym, Carrageenase, Perhydrolase, Oxidase, Oxidoreduktase oder einer Lipase.

Alle Sachverhalte, Gegenstände und Ausführungsformen, die vorstehend bereits beschrieben sind, sind auch auf diese Erfindungsgegenstände anwendbar. Daher wird an dieser Stelle ausdrücklich auf die Offenbarung an entsprechender Stelle verwiesen mit dem Hinweis, dass diese Offenbarung auch für die erfindungsgemäßen Verwendungen gilt (Verwendung des Vektors bzw. der Wirtszelle).

Beispiele:
Alle molekularbiologischen Arbeitsschritte folgen Standardmethoden, wie sie beispielsweise in dem Handbuch von Fritsch, Sambrook und Maniatis "Molecular cloning: a laboratory manual", Cold Spring Harbour Laboratory Press, New York, 1989, oder vergleichbaren einschlägigen Werken angegeben sind. Enzyme und Baukästen (Kits) wurden nach den Angaben der jeweiligen Hersteller eingesetzt.

### Beispiel 1: Herstellung erfindungsgemäßer Expressionsvektoren

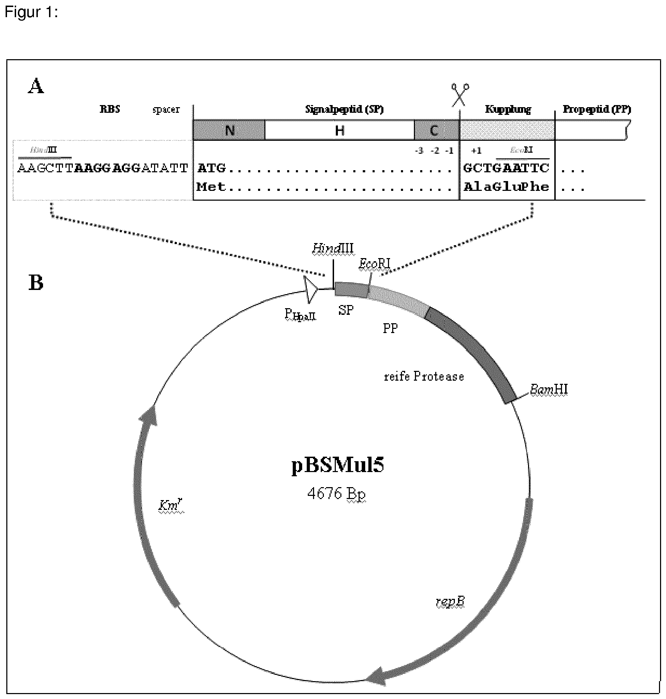

Das Plasmid pBSMuL3 (Brockmeier *et al.*, 2006) wurde durch Sacl-Restriktion und anschließende Religation um den E.coli-Anteil verkürzt. Das resultierende Plasmid, pBSMuL5 (vgl. Figur 1) diente als Vektor zur Klonierung der Proteasen inklusive Propeptid in die Schnittstellen EcoRI und BamHI. Dazu wurden die Gene der Protease gemäß SEQ ID NO. 8 mit den Primern gemäß SEQ ID NO. 11 und SEQ ID NO. 12, und der alkalischen Protease gemäß SEQ ID NO. 9 mit den Primern gemäß SEQ ID NO. 13 und SEQ ID NO. 14 amplifiziert. Die resultierenden Plasmide dienten als Vektoren zur Klonierung der Signalpeptide in die Schnittstellen HindIII und EcoRI. Das DNA-Fragment des Kontroll-Signalpeptids SubC (B. *licheniformis,* NCBI ("National Center for Biotechnology Information") Zugriffsnummer ("Accession Number"): X91260.1) als "Benchmark" wurde mit Hilfe der Primer gemäß SEQ ID NO. 15 und SEQ ID NO. 16 amplifiziert und jeweils in die HindIII und EcoRI-Schnittstellen der Plasmide kloniert, so dass Plasmide mit einer Nukleinsäuresequenz b) entstanden, die für ein Protein mit dem Signalpeptid SubC in Verbindung mit einer Protease gemäß SEQ ID NO. 8 (Plasmid 1) bzw. SEQ ID NO. 9 (Plasmid 2) codieren. Diese Plasmide dienten nachfolgend als Kontrolle bzw. "Benchmark". Das DNA-Fragment des Signalpeptids gemäß SEQ ID NO. 2 wurde mit Hilfe der Primer gemäß SEQ ID NO. 19 und SEQ ID NO. 20, das DNA-Fragment des Signalpeptids gemäß SEQ ID NO. 4 mit den Primern gemäß SEQ ID NO. 17 und SEQ ID NO. 18, und das DNA-Fragment des Signalpeptids gemäß SEQ ID NO. 6 mit den Primern gemäß SEQ ID NO. 21 und SEQ ID NO. 22 amplifiziert. Während die DNA-Fragmente der Signalpeptide gemäß SEQ ID NO. 2 und 4 jeweils in den Vektor kloniert wurden, der für eine Protease gemäß SEQ ID NO. 8 codiert (Plasmide 3 und 4), wurde das DNA-Fragment des Signalpeptids gemäß SEQ ID NO. 6 in den Vektor eingefügt, der für eine Protease gemäß SEQ ID NO. 9 codiert (Plasmid 5). Klonierungsbedingt wurde dabei zwischen der DNA-Sequenz des jeweiligen Signalpeptids und der DNA-Sequenz des Propeptids der jeweiligen Protease eine Sequenz von 9 Nukleotiden eingeführt, die für die Aminosäurefolge AEF kodiert (vgl. Figur 1). Diese so genannte Verbindungssequenz beinhaltet die Erkennungssequenz der Restriktionsendonuklease EcoRI.

Alle als Primer verwendeten Oligonukleotide sind in nachfolgender Tabelle 1 aufgelistet:

**Tabelle 1:**

| Bezeichnung | Nukleotidsequenz (in 5'→ 3' Orientierung; die Unterstreichungen kennzeichnen die Restriktionsschnittstelle) | Schnittstelle |
|---|---|---|
| SEQ ID NO. 11 | ATATGAATTCGCTGAGGAAGCAAAAGAAAA | EcoRI |
| SEQ ID NO. 12 | ATATGGATC**C**TTAGCGTGTTGCCGCTTCTGC | BamHI |
| SEQ ID NO. 13 | ATATGAATTCGCTGAGGAAGCAAAAGAAAA | EcoRI |
| SEQ ID NO. 14 | ATATGGATCCTTAGCGCGTTGCTGCATCTGC | BamHI |
| SEQ ID NO. 15 | ATATAAGCTTAAGGAGGATATT*AT*GATGAGGAAAAAGAGT TTT | HindIII |
| SEQ ID NO. 16 | ATATGAATTCAGCTGCAGAAGCGGAATCGCTGAA | EcoRI |
| SEQ ID NO. 17 | ATATAAGCTTAAGGAGGATATTATGAAAAAACTATTCAAAA CC | HindIII |
| SEQ ID NO. 18 | ATATGAATTCAGCAGCCGCCGCAGATTGTGAGAA | EcoRI |
| SEQ ID NO. 19 | ATATAAGCTTAAGGAGGATATTATGGCGAAACCACTATCA AAA | HindIII |
| SEQ ID NO. 20 | ATATGAATTCAGCAGCGTCTGCCGCGGGTAAACC | EcoRI |
| SEQ ID NO. 21 | ATATAAGCTTAAGGAGGATATTATGACATTGACTAAACTG AAA | HindIII |
| SEQ ID NO. 22 | ATATGAATTCAGCGGCAAGTGCCTGACTGGAAAA | EcoRI |

### Beispiel 2: Expression der Proteine

Ein *Bacillus licheniformis*-Stamm wurden mit den Plasmiden 1 bis 5 transformiert, um die verschiedenen Protease-Produktionsstämme zu erhalten. Für die Inokulation von Kulturen wurden Einzelkolonien von Agarplatten verwendet, die über Nacht (ÜN) inkubiert wurden. Für die quantitative Bestimmung der Sekretionseffizienz wurden die Einzelkolonien direkt von den Agarplatten in Deepwell-MTP (Mikrotiterplatte; 96 Kavitäten mit je 1 mL selektiven LB-Medium) überführt. Dabei wurde jede Einzelkolonie parallel in mindestens zwei Kavitäten überführt, um eine Doppel- oder Dreifach-Bestimmung durch die Mehrfach-Anzucht des jeweiligen Klons zu erhalten.

Für das Beimpfen der Deepwell-MTP wurden ausschließlich Klone verwendet, die über Nacht bei 37°C inkubiert wurden. Nach der Anzucht für 20 h bei 37°C im Mikrotiterplattenschüttler (Timix 5, Firma Edmund-Bühler, Hechingen) wurden alle Klone auf LB-Agarplatten repliziert und anschließend die Zellen mittels Zentrifugation sedimentiert (4000 UpM, 20 min, 4°C). Alle folgenden Pipettierschritte wurden mit Hilfe von Mehrkanalpipetten (Eppendorf, Hamburg) durchgeführt, wobei der "reverse pipetting"-Modus verwendet wurde und keine Volumina kleiner als 15 µl pipettiert wurden. Es wurde jeweils das kleinste Volumen in der MTP vorgelegt und die größeren Volumina dazu gegeben und die MTP bei jedem Verdünnungsschritt 10 Sekunden im Spektralphotometer "Spektramax 250" (Molecular Devices, Sunnyvale, USA) gemischt. Für die Erstellung der entsprechenden Verdünnungen wurde der Kulturüberstand per Mehrkanalpipette abgenommen und in Mikrotiterplatten (96 Kavitäten, F-Boden, glasklar, Firma Greiner Bio-One, Frickenhausen) überführt.

Anschließend wurde die proteolytische Aktivität in den Kulturüberständen bzw. Verdünnungen über die Freisetzung des Chromophors para-Nitroanilin (pNA) aus dem Substrat suc-L-Ala-L-Ala-L-Pro-L-Phe-p-Nitroanilid (suc-AAPF-pNA) bestimmt. Die Protease spaltet das Substrat und setzt pNA frei. Die Freisetzung des pNA verursacht eine Zunahme der Extinktion bei 410 nm, deren zeitlicher Verlauf ein Maß für die enzymatische Aktivität ist (vgl. Del Mar et al., Anal.Biochem., 99: 316-320, 1979).

Für die Ermittlung der Sekretionseffizienz der verschiedenen Stämme wurde in jeder MTP-Anzucht ein internes Kontroll-Konstrukt Plasmid 1 bzw. Plasmid 2) mit angezogen. Die im Kulturüberstand ermittelte proteolytische Aktivität des Stammes mit dem Kontroll-Konstrukt wurde als 100 % definiert.

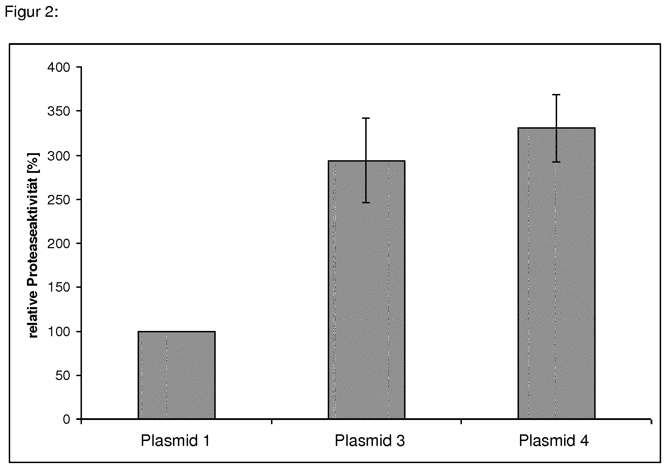

Die Stämme mit den erfindungsgemäßen Plasmiden 3 und 4 erreichten verglichen mit der Kontrolle, die das Plasmid 1 enthielt, eine um 194% +/- 48 bzw. 230% +/- 38 erhöhte Proteaseaktivität (vgl. Figur 2).

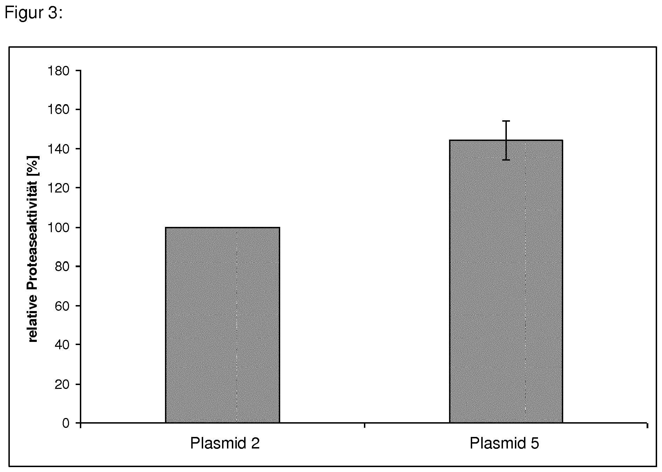

Der Stamm mit dem erfindungsgemäßen Plasmid 5 erreichte verglichen mit der Kontrolle, die das Plasmid 2 enthielt, eine um 44% +/- 10 erhöhte Proteaseaktivität (vgl. Figur 3).

### Beschreibung der Figuren

- Figur 1:: Schema der Klonierungsstrategie im Bacillus-Expressionsvektor pBSMul5 (modifiziert nach Brockmeier et al., 2006). (A) Die DNA-Fragmente der Signalpeptide wurden am N-Terminus mit einer Hindlll-Restriktionsschnittstelle, einer standardisierten Ribosomenbindestelle (RBS), gefolgt von einer Spacer-Region und dem standardisierten Startcodon für Methionin amplifiziert. Zwischen Signalpeptid und N-Terminus der zu sezernierenden Protease wurde eine Kupplung mit einem Alanin an der ,,+1"-Position und der EcoRI-Restriktionsschnittstelle angefügt. (B) Bacillus-Vektor pBSMul5 mit dem Hpall-Promotor, dem jeweiligen Sekretionstarget (kloniert über EcoRI und BamHI) sowie der Kanamycin-Resistenz-Kassette und dem Replikationsprotein repB für Bacillus.
- Figur 2:: Relative Proteaseaktivität im Kulturüberstand von Bacillus licheniformis mit der Protease gemäß SEQ ID NO. 8 und drei verschiedenen Signalpeptiden in pBSMul5. Die proteolytische Aktivität von dem Konstrukt Plasmid 1 wurde als 100% definiert (Kontrolle). Die Werte wurden in mindestens zwei voneinander unabhängigen Anzuchten ermittelt. Die Fehlerbalken geben die Standardabweichung an.
- Figur 3:: Relative Proteaseaktivität im Kulturüberstand von Bacillus licheniformis mit der Protease gemäß SEQ ID NO. 9 und zwei verschiedenen Signalpeptiden in pBSMul5. Die proteolytische Aktivität von dem Konstrukt Plasmid 2 wurde als 100% definiert (Kontrolle). Die Werte wurden in mindestens zwei voneinander unabhängigen Anzuchten ermittelt. Die Fehlerbalken geben die Standardabweichung an.

## Patentansprüche

1. Expressionsvektor umfassend
a) eine Promotorsequenz und
b) eine Nukleinsäuresequenz, die für ein Protein codiert, wobei das Protein ein Signalpeptid und eine weitere Aminosäuresequenz umfasst und das Signalpeptid eine Aminosäuresequenz umfasst, die zu der in SEQ ID NO. 4 angegebenen Aminosäuresequenz zu mindestens 80% identisch ist, **dadurch gekennzeichnet, dass** die weitere Aminosäuresequenz des Proteins die Aminosäuresequenz einer Protease, Amylase, Cellulase, Hemicellulase, Mannanase, Tannase, Xylanase, Xanthanase, Xyloglucanase, ß-Glucosidase, Pektin-spaltendes Enzym, Carrageenase, Perhydrolase, Oxidase, Oxidoreduktase oder einer Lipase umfasst.

2. Expressionsvektor nach Anspruch 1, **dadurch gekennzeichnet, dass** das von der Nukleinsäuresequenz b) codierte Signalpeptid eine Aminosäuresequenz gemäß SEQ ID NO. 4 aufweist.

3. Expressionsvektor nach einem der Ansprüche 1 oder 2, **dadurch gekennzeichnet, dass** das Signalpeptid N-terminal von der weiteren Aminosäuresequenz in dem von der Nukleinsäuresequenz b) codierten Protein angeordnet ist.

4. Expressionsvektor nach einem der Ansprüche 1 bis 3, **dadurch gekennzeichnet, dass** das von der Nukleinsäuresequenz b) codierte Protein ferner eine Verbindungssequenz umfasst, die zwischen dem Signalpeptid und der weiteren Aminosäuresequenz des Proteins angeordnet ist, insbesondere wobei die Verbindungssequenz eine Länge zwischen 1 und 50 Aminosäuren aufweist.

5. Expressionsvektor nach einem der Ansprüche 1 bis 4, **dadurch gekennzeichnet, dass** die weitere Aminosäuresequenz des Proteins die Aminosäuresequenz einer Protease umfasst, wobei die Aminosäuresequenz der Protease
mindestens zu 80% identisch zu SEQ ID NO. 7 ist, oder
mindestens zu 80% identisch zu SEQ ID NO. 8 ist, oder
mindestens zu 80% identisch zu SEQ ID NO. 9 ist, oder
mindestens zu 80% identisch zu SEQ ID NO. 10 ist und an Position 99 in der Zählung gemäß SEQ ID NO. 10 die Aminosäure Glutaminsäure (E) oder Asparaginsäure (D) aufweist, oder mindestens zu 80% identisch zu SEQ ID NO. 10 ist und an Position 99 in der Zählung gemäß SEQ ID NO. 10 die Aminosäure Glutaminsäure (E) oder Asparaginsäure (D) aufweist und ferner in der Zählung gemäß SEQ ID NO. 10 mindestens eine der folgenden Aminosäuren aufweist:
(a) Threonin an Position 3 (3T),
(b) Isoleucin an Position 4 (4I),
(c) Alanin, Threonin oder Arginin an Position 61 (61A, 61T oder 61R),
(d) Asparaginsäure oder Glutaminsäure an Position 154 (154D oder 154E),
(e) Prolin an Position 188 (188P),
(f) Methionin an Position 193 (193M),
(g) Isoleucin an Position 199 (199I),
(h) Asparaginsäure, Glutaminsäure oder Glycin an Position 211 (211D, 211E oder 211G),
(i) Kombinationen der Aminosäuren (a) bis (h).

6. Nicht menschliche Wirtszelle enthaltend einen Expressionsvektor gemäß einem der Ansprüche 1 bis 5.

7. Wirtszelle nach Anspruch 6, **dadurch gekennzeichnet, dass** sie ein Bakterium ist, bevorzugt eines, das ausgewählt ist aus der Gruppe der Gattungen von Escherichia, Klebsiella, Bacillus, Staphylococcus, Corynebakterium, Arthrobacter, Streptomyces, Stenotrophomonas und Pseudomonas, weiter bevorzugt eines, das ausgewählt ist aus der Gruppe von Escherichia coli, Klebsiella planticola, Bacillus licheniformis, Bacillus lentus, Bacillus amyloliquefaciens, Bacillus subtilis, Bacillus alcalophilus, Bacillus globigii, Bacillus gibsonii, Bacillus clausii, Bacillus halodurans, Bacillus pumilus, Staphylococcus carnosus, Corynebacterium glutamicum, Arthrobacter oxidans, Streptomyces lividans, Streptomyces coelicolor und Stenotrophomonas maltophilia, insbesondere Bacillus licheniformis.

8. Verfahren zur Herstellung eines Proteins umfassend die Verfahrensschritte
(a) Kultivieren einer Wirtszelle gemäß einem der Ansprüche 6 oder 7
(b) Isolieren des Proteins aus dem Kulturmedium oder aus der Wirtszelle.

9. Verwendung eines Expressionsvektors gemäß einem der Ansprüche 1 bis 5 oder einer Wirtszelle gemäß einem der Ansprüche 6 oder 7 zur Herstellung eines Proteins.

## Claims

1. An expression vector comprising
a) a promoter sequence and
b) a nucleic acid sequence which encodes a protein, the protein comprising a signal peptide and a further amino acid sequence and the signal peptide comprising an amino acid sequence which is at least 80% identical to the amino acid sequence specified in SEQ ID NO. 4, wherein the further amino acid sequence of the protein comprises the amino acid sequence of a protease, amylase, cellulase, hemicellulase, mannanase, tannase, xylanase, xanthanase, xyloglucanase, β-glucosidase, pectin-cleaving enzyme, carrageenase, perhydrolase, oxidase, oxidoreductase or a lipase.

2. The expression vector according to claim 1, wherein the signal peptide encoded by the nucleic acid sequence b) has an amino acid sequence according to SEQ ID NO. 4.

3. The expression vector according to either of claims 1 and 2, wherein the signal peptide is arranged N-terminal to the further amino acid sequence in the protein encoded by the nucleic acid sequence b).

4. The expression vector according to any of claims 1 to 3, wherein the protein encoded by the nucleic acid sequence b) further comprises a connecting sequence arranged between the signal peptide and the further amino acid sequence of the protein, the length of the connecting sequence being in particular between 1 and 50 amino acids.

5. The expression vector according to any of claims 1 to 4, wherein the further amino acid sequence of the protein comprises the amino acid sequence of a protease, the amino acid sequence of the protease being
at least 80% identical to SEQ ID NO. 7, or being
at least 80% identical to SEQ ID NO. 8, or being
at least 80% identical to SEQ ID NO. 9, or being
at least 80% identical to SEQ ID NO. 10 and having the amino acid glutamic acid (E) or aspartic acid (D) at position 99 in the numbering according to SEQ ID NO. 10, or being
at least 80% identical to SEQ ID NO. 10 and having the amino acid glutamic acid (E) or aspartic acid (D) at position 99 in the numbering according to SEQ ID NO. 10 and having, furthermore, at least one of the following amino acids in the numbering according to SEQ ID NO. 10:
(a) threonine at position 3 (3T),
(b) isoleucine at position 4 (4I),
(c) alanine, threonine or arginine at position 61 (61A, 61T or 61R),
(d) aspartic acid or glutamic acid at position 154 (154D or 154E),
(e) proline at position 188 (188P),
(f) methionine at position 193 (193M),
(g) isoleucine at position 199 (199I),
(h) aspartic acid, glutamic acid or glycine at position 211 (211D, 211E or 211G),
(i) combinations of the amino acids (a) to (h).

6. A nonhuman host cell comprising an expression vector according to any of claims 1 to 5.

7. The host cell according to claim 6, wherein it is a bacterium, preferably one selected from the group of the genera of Escherichia, Klebsiella, Bacillus, Staphylococcus, Corynebacterium, Arthrobacter, Streptomyces, Stenotrophomonas and Pseudomonas, more preferably one selected from the group of Escherichia coli, Klebsiella planticola, Bacillus licheniformis, Bacillus lentus, Bacillus amyloliquefaciens, Bacillus subtilis, Bacillus alcalophilus, Bacillus globigii, Bacillus gibsonii, Bacillus clausii, Bacillus halodurans, Bacillus pumilus, Staphylococcus carnosus, Corynebacterium glutamicum, Arthrobacter oxidans, Streptomyces lividans, Streptomyces coelicolor and Stenotrophomonas maltophilia, more particularly Bacillus licheniformis.

8. A method for preparing a protein, comprising the method steps of
(a) culturing a host cell according to either of claims 6 and 7
(b) isolating the protein from the culture medium or from the host cell.

9. The use of an expression vector according to any of claims 1 to 5 or of a host cell according to either of claims 6 and 7 for preparing a protein.

## Revendications

1. Vecteur d'expression comprenant :
a) une séquence promotrice et
b) une séquence d'acide nucléique qui code pour une protéine, la protéine comprenant un peptide signal et une séquence d'acides aminés supplémentaire et le peptide signal comprenant une séquence d'acides aminés qui présente une identité d'au moins 80 % avec la séquence d'acides aminés présentée dans la SEQ ID NO: 4, **caractérisé en ce que** la séquence d'acides aminés supplémentaire de la protéine comprend la séquence d'acides aminés d'une protéase, d'une amylase, d'une cellulase, d'une hémicellulase, d'une mannanase, d'une tannase, d'une xylanase, d'une xanthanase, d'une xyloglucanase, d'une β-glucosidase, d'une enzyme de dissociation de la pectine, d'une carragénase, d'une perhydrolase, d'une oxydase, d'une oxydoréductase ou d'une lipase.

2. Vecteur d'expression selon la revendication 1, **caractérisé en ce que** le peptide signal codé par la séquence d'acide nucléique b) présente une séquence d'acides aminés selon la SEQ ID NO: 4.

3. Vecteur d'expression selon l'une des revendications 1 ou 2, **caractérisé en ce que** le peptide signal est disposé en position N-terminale de la séquence d'acides aminés supplémentaire dans la protéine codée par la séquence d'acide nucléique b).

4. Vecteur d'expression selon l'une des revendications 1 à 3, **caractérisé en ce que** la protéine codée par la séquence d'acide nucléique b) comprend en outre une séquence de liaison, qui est disposée entre le peptide signal et la séquence d'acides aminés supplémentaire de la protéine, en particulier la séquence de liaison présentant une longueur entre 1 et 50 acides aminés.

5. Vecteur d'expression selon l'une des revendications 1 à 4, **caractérisé en ce que** la séquence d'acides aminés supplémentaire de la protéine comprend la séquence d'acides aminés d'une protéase, la séquence d'acides aminés de la protéase
ayant une identité d'au moins 80 % avec la SEQ ID NO: 7, ou
ayant une identité d'au moins 80 % avec la SEQ ID NO: 8 ou
ayant une identité d'au moins 80 % avec la SEQ ID NO: 9 ou
ayant une identité d'au moins 80 % avec la SEQ ID NO: 10, et présentant en position 99 dans la numérotation selon la SEQ ID NO: 10 l'acide aminé acide glutamique (E) ou acide asparagique (D), ou ayant une identité d'au moins 80 % avec la SEQ ID NO: 10 et présentant en position 99 dans la numérotation selon la SEQ ID NO: 10 l'acide aminé acide glutamique (E) ou acide asparagique (D), et en outre dans la numérotation selon la SEQ ID NO: 10 présentant au moins l'un des acides aminés suivants :
(a) thréonine en position 3 (3T),
(b) isoleucine en position 4 (4I),
(c) alanine, thréonine ou arginine en position 61 (61A, 61T ou 61R),
(d) acide asparagique ou acide glutamique en position 154 (154D ou 154E),
(e) proline en position 188 (188P),
(f) méthionine en position 193 (193M),
(g) isoleucine en position 199 (199I),
(h) acide asparagique, acide glutamique ou glycine en position 211 (211D, 211E ou 211G),
(i) les combinaisons des acides aminés (a) à (h).

6. Cellule hôte non humaine contenant un vecteur d'expression selon l'une des revendications 1 à 5.

7. Cellule hôte selon la revendication 6, **caractérisée en ce qu'**elle est une bactérie, de préférence une bactérie qui est choisie dans le groupe des genres Escherichia, Klebsiella, Bacillus, Staphylococcus, Corynebacterium, Arthrobacter, Streptomyces, Stenotrophomonas et Pseudomonas, plus précisément une bactérie qui est choisie dans le groupe Escherichia coli, Klebsiella planticola, Bacillus licheniformis, Bacillus lentus, Bacillus amyloliquefaciens, Bacillus subtilis, Bacillus alcalophilus, Bacillus globigii, Bacillus gibsonii, Bacillus clausii, Bacillus halodurans, Bacillus pumilus, Staphylococcus camosus, Corynebacterium glutamicum, Arthrobacter oxydans, Streptomyces lividans, Streptomyces coelicolor et Stenotrophomonas maltophilia, en particulier Bacillus licheniformis.

8. Procédé de fabrication d'une protéine comprenant les étapes :
(a) culture d'une cellule hôte selon l'une des revendications 6 ou 7
(b) isolement de la protéine à partir du milieu de culture et de la cellule hôte.

9. Utilisation d'un vecteur d'expression selon l'une des revendications 1 à 5 ou d'une cellule hôte selon l'une des revendications 6 ou 7 pour fabriquer une protéine.
